(19)

**Europäisches Patentamt**
**European Patent Office**
**Office européen des brevets**

(11) **EP 4 114 980 B1**

(12) **EUROPEAN PATENT SPECIFICATION**

(45) Date of publication and mention
of the grant of the patent:
**26.11.2025 Bulletin 2025/48**

(21) Application number: **21708224.7**

(22) Date of filing: **01.03.2021**

(51) International Patent Classification (IPC):
***C12Q 1/6886*** *(2018.01)*

(52) Cooperative Patent Classification (CPC):
**C12Q 1/6886;** C12Q 2600/106; C12Q 2600/158

(86) International application number:
**PCT/EP2021/054955**

(87) International publication number:
**WO 2021/175746 (10.09.2021 Gazette 2021/36)**

(54) **PREDICTION OF RADIOTHERAPY RESPONSE FOR PROSTATE CANCER SUBJECT BASED ON IMMUNE DEFENSE RESPONSE GENES**

VORHERSAGE DER RADIOTHERAPIEREAKTION FÜR PROSTATAKREBSPATIENTEN AUF DER BASIS VON IMMUNABWEHR-ANTWORTGENEN

PRÉDICTION DE LA RÉPONSE À LA RADIOTHÉRAPIE POUR UN SUJET ATTEINT D'UN CANCER DE LA PROSTATE SUR LA BASE DES GÈNES DE LA RÉPONSE DE DÉFENSE IMMUNITAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **05.03.2020 EP 20161176**

(43) Date of publication of application:
**11.01.2023 Bulletin 2023/02**

(73) Proprietor: **Koninklijke Philips N.V.**
**5656 AG Eindhoven (NL)**

(72) Inventors:
• **HOFFMANN, Ralf, Dieter**
**5656 AE Eindhoven (NL)**
• **ORSEL, Joukje, Garrelina**
**5656 AE Eindhoven (NL)**
• **DE KLERK - STARMANS, Maud**
**5656 AE Eindhoven (NL)**
• **VAN LIESHOUT, Ron, Martinus, Laurentius**
**5656 AE Eindhoven (NL)**

(74) Representative: **Philips Intellectual Property & Standards**
**High Tech Campus 52**
**5656 AG Eindhoven (NL)**

(56) References cited:
**WO-A2-2019/028285     US-B2- 7 892 740**

• **ZHAO SHUANG G ET AL: "Development and validation of a 24-gene predictor of response to postoperative radiotherapy in prostate cancer: a matched, retrospective analysis", THE LANCET ONCOLOGY, ELSEVIER, AMSTERDAM, NL, vol. 17, no. 11, 12 October 2016 (2016-10-12), pages 1612 - 1620, XP029794201, ISSN: 1470-2045, DOI: 10.1016/S1470-2045(16)30491-0**

Remarks:
The complete document including Reference Table(s) and the Sequence Listing(s) can be downloaded from the EPO website

**Description**

FIELD OF THE INVENTION

The invention relates to a method of predicting a response of a prostate cancer subject to radiotherapy. Moreover, the invention relates to a diagnostic kit, to a use of the kit in a method of predicting a response of a prostate cancer subject to radiotherapy, to a use of a gene expression profile for each of five or more immune defense response genes in radiotherapy prediction for a prostate cancer subject, and to a corresponding computer program product.

BACKGROUND OF THE INVENTION

Cancer is a class of diseases in which a group of cells displays uncontrolled growth, invasion and sometimes metastasis. These three malignant properties of cancers differentiate them from benign tumours, which are self-limited and do not invade or metastasize. Prostate Cancer (PCa) is the second most commonly-occurring non-skin malignancy in men, with an estimated 1.3 million new cases diagnosed and 360,000 deaths world-wide in 2018 (see Bray F. et al., "Global cancer statistics 2018: GLOBOCAN estimates of incidence and mortality worldwide for 36 cancers in 185 countries", CA Cancer J Clin, Vol. 68, No. 6, pages 394-424, 2018). In the US, about 90% of the new cases concern localized cancer, meaning that metastases have not yet been formed (see ACS (American Cancer Society), "Cancer Facts & Figures 2010", 2010).

For the treatment of primary localized prostate cancer, several radical therapies are available, of which surgery (radical prostatectomy, RP) and radiation therapy (RT) are most commonly used. RT is administered via an external beam or via the implantation of radioactive seeds into the prostate (brachytherapy) or a combination of both. It is especially preferable for patients who are not eligible for surgery or have been diagnosed with a tumour in an advanced localized or regional stage. Radical RT is provided to up to 50% of patients diagnosed with localized prostate cancer in the US (see ACS, 2010, ibid).

After treatment, prostate cancer antigen (PSA) levels in the blood are measured for disease monitoring. An increase of the blood PSA level provides a biochemical surrogate measure for cancer recurrence or progression. However, the variation in reported biochemical progression-free survival (bPFS) is large (see Grimm P. et al., "Comparative analysis of prostate-specific antigen free survival outcomes for patients with low, intermediate and high risk prostate cancer treatment by radical therapy. Results from the Prostate Cancer Results Study Group", BJU Int, Suppl. 1, pages 22-29, 2012). For many patients, the bPFS at 5 or even 10 years after radical RT may lie above 90%. Unfortunately, for the group of patients at medium and especially higher risk of recurrence, the bPFS can drop to around 40% at 5 years, depending on the type of RT used (see Grimm P. et al., 2012, ibid).

A large number of the patients with primary localized prostate cancer that are not treated with RT will undergo RP (see ACS, 2010, ibid). After RP, an average of 60% of patients in the highest risk group experience biochemical recurrence after 5 and 10 years (see Grimm P. et al., 2012, ibid). In case of biochemical progression after RP, one of the main challenges is the uncertainty whether this is due to recurring localized disease, one or more metastases or even an indolent disease that will not lead to clinical disease progression (see Dal Pra A. et al., "Contemporary role of postoperative radiotherapy for prostate cancer", Transl Androl Urol, Vo. 7, No. 3, pages 399-413, 2018, and Herrera F.G. and Berthold D.R., "Radiation therapy after radical prostatectomy: Implications for clinicians", Front Oncol, Vol. 6, No. 117, 2016). RT to eradicate remaining cancer cells in the prostate bed is one of the main treatment options to salvage survival after a PSA increase following RP. The effectiveness of salvage radiotherapy (SRT) results in 5-year bPFS for 18% to 90% of patients, depending on multiple factors (see Herrera F.G. and Berthold D.R., 2016, ibid, and Pisansky T.M. et al., "Salvage radiation therapy dose response for biochemical failure of prostate cancer after prostatectomy - A multi-institutional observational study", Int J Radiat Oncol Biol Phys, Vol. 96, No. 5, pages 1046-1053, 2016).

It is clear that for certain patient groups, radical or salvage RT is not effective. Their situation is even worsened by the serious side effects that RT can cause, such as bowel inflammation and dysfunction, urinary incontinence and erectile dysfunction (see Resnick M.J. et al., "Long-term functional outcomes after treatment for localized prostate cancer", N Engl J Med, Vol. 368, No. 5, pages 436-445, 2013, and Hegarty S.E. et al., "Radiation therapy after radical prostatectomy for prostate cancer: Evaluation of complications and influence of radiation timing on outcomes in a large, population-based cohort", PLoS One, Vol. 10, No. 2, 2015). In addition, the median cost of one course of RT based on Medicare reimbursement is $ 18,000, with a wide variation up to about $ 40,000 (see Paravati A.J. et al., "Variation in the cost of radiation therapy among medicare patients with cancer", J Oncol Pract, Vol. 11, No. 5, pages 403-409, 2015). These figures do not include the considerable longitudinal costs of follow-up care after radical and salvage RT.

An improved prediction of effectiveness of RT for each patient, be it in the radical or the salvage setting, would improve therapy selection and potentially survival. This can be achieved by 1) optimizing RT for those patients where RT is predicted to be effective (e.g., by dose escalation or a different starting time) and 2) guiding patients where RT is predicted not to be effective to an alternative, potentially more effective form of treatment. Further, this would reduce suffering for those patients who would be spared ineffective therapy and would reduce costs spent on ineffective therapies.

Numerous investigations have been conducted into measures for response prediction of radical RT (see Hall W.A. et al., "Biomarkers of outcome in patients with localized prostate cancer treated with radiotherapy", Semin Radiat Oncol, Vol. 27, pages 11-20, 2016, and Raymond E. et al., "An appraisal of analytical tools used in predicting clinical outcomes following radiation therapy treatment of men with prostate cancer: A systematic review", Radiat Oncol, Vol. 12, No. 1, page 56, 2017) and SRT (see Herrera F.G. and Berthold D.R., 2016, ibid). Many of these measures depend on the concentration of the blood-based biomarker PSA. Metrics investigated for prediction of response before start of RT (radical as well as salvage) include the absolute value of the PSA concentration, its absolute value relative to the prostate volume, the absolute increase over a certain time and the doubling time. Other frequently considered factors are the Gleason score and the clinical tumour stage. For the SRT setting, additional factors are relevant, e.g., surgical margin status, time to recurrence after RP, pre-/peri-surgical PSA values and clinico-pathological parameters.

Although these clinical variables provide limited improvements in patient stratification in various risk groups, there is a need for better predictive tools.

A wide range of biomarker candidates in tissue and bodily fluids has been investigated, but validation is often limited and generally demonstrates prognostic information and not a predictive (therapy-specific) value (see Hall W.A. et al., 2016, ibid). A small number of gene expression panels is currently being validated by commercial organizations. One or a few of these may show predictive value for RT in future (see Dal Pra A. et al., 2018, ibid).

In conclusion, a strong need for better prediction of response to RT remains, for primary prostate cancer as well as for the post-surgery setting.

Zhao S.G. et al., "Development and validation of a 24-gene predictor of response to postoperative radiotherapy in prostate cancer: a matched, retrospective analysis", The Lancet Oncology, Vol. 17, No. 11, pages 1612-1620, 2016, starts out from the premise that postoperative radiotherapy has an important role in the treatment of prostate cancer, but that personalised patient selection could improve outcomes and spare unnecessary toxicity. The document aims at developing and validating a gene expression signature to predict which patients would benefit most from postoperative radiotherapy.

WO 2019/028285 A2 discloses methods, systems, and kits for the diagnosis, prognosis and the determination of cancer progression of prostate cancer in a subject. In particular, the disclosure relates to the use of immune cell-specific gene expression in determining prognosis and identifying individuals in need of treatment for prostate cancer who will be responsive to radiation therapy.

## SUMMARY OF THE INVENTION

It is an objective of the invention to provide a method of predicting a response of a prostate cancer subject to radiotherapy, which allows to make better treatment decisions. It is a further objective of the invention to provide a diagnostic kit, a use of the kit in a method of predicting a response of a prostate cancer subject to radiotherapy, a use of a gene expression profile for each of five or more immune defense response genes in radiotherapy prediction for a prostate cancer subject, and a corresponding computer program product.

In a first aspect of the present invention, a method of predicting a response of a prostate cancer subject to radiotherapy is presented, comprising:

- determining or receiving the result of a determination of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
- determining, preferably by a processor, the prediction of the radiotherapy response based on the gene expression profiles for the five or more immune defense response genes, and
- optionally, providing the prediction or a therapy recommendation based on the prediction to a medical caregiver or the subject.

In recent years, the importance of the immune system in cancer inhibition as well as in cancer initiation, promotion and metastasis has become very evident (see Mantovani A. et al., "Cancer-related inflammation", Nature, Vol. 454, No. 7203, pages 436-444, 2008, and Giraldo N.A. et al., "The clinical role of the TME in solid cancer", Br J Cancer, Vol. 120, No. 1, pages 45-53, 2019). The immune cells and the molecules they secrete form a crucial part of the tumour microenvironment and most immune cells can infiltrate the tumour tissue. The immune system and the tumour affect and shape one another. Thus, anti-tumour immunity can prevent tumour formation while an inflammatory tumour environment may promote cancer initiation and proliferation. At the same time, tumour cells that may have originated in an immune system-independent manner will shape the immune microenvironment by recruiting immune cells and can have a pro-inflammatory effect while also suppressing anti-cancer immunity.

Some of the immune cells in the tumour microenvironment will have either a general tumour-promoting or a general tumour-inhibiting effect, while other immune cells exhibit plasticity and show both tumour-promoting and tumour-inhibiting potential. Thus, the overall immune microenvironment of the tumour is a mixture of the various immune cells present, the cytokines they produce and their interactions with tumour cells and with other cells in the tumour microenvironment (see Giraldo N.A. et al., 2019, ibid).

The principles described above with regard to the role of the immune system in cancer in general also apply to prostate cancer. Chronic inflammation has been linked to the formation of benign as well as malignant prostate tissue (see Hall W.A. et al., 2016, ibid) and most prostate cancer tissue samples show immune cell infiltrates. The presence of specific immune cells with a pro-tumour effect has been correlated with worse prognosis, while tumours in which natural killer cells were more activated showed better response to therapy and longer recurrence-free periods (see Shiao S.L. et al., "Regulation of prostate cancer progression by tumor microenvironment", Cancer Lett, Vol. 380, No. 1, pages 340-348, 2016).

While a therapy will be influenced by the immune components of the tumour microenvironment, RT itself extensively affects the make-up of these components (see Barker H.E. et al., "The tumor microenvironment after radiotherapy: Mechanisms of resistance or recurrence", Nat Rev Cancer, Vol. 15, No. 7, pages 409-425, 2015). Because suppressive cell types are comparably radiation-insensitive, their relative numbers will increase. Counteractively, the inflicted radiation damage activates cell survival pathways and stimulates the immune system, triggering inflammatory responses and immune cell recruitment. Whether the net effect will be tumour-promoting or tumour-suppressing is as yet uncertain, but its potential for enhancement of cancer immunotherapies is being investigated.

The present invention is based on the idea that, since the status of the immune system and of the immune microenvironment have an impact on therapy effectiveness, the ability to identify markers predictive for this effect might help to be better able to predict overall RT response.

The integrity and stability of genomics DNA is permanently under stress induced by various cell internal and external factors like exposure to radiation, viral or bacterial infections, but also oxidation and replication stress (see Gasser S. et al., "Sensing of dangerous DNA", Mechanisms of Aging and Development, Vol. 165, pages 33-46, 2017). In order to maintain DNA structure and stability, a cell must be able to recognize all types of DNA damages like single or double strand breaks etc. induced by various factors. This process involves the participation of a multitude of specific proteins depending on the kind of damage as part of DNA recognition pathways.

Recent evidence suggests that mis-localized DNA (e.g., DNA unnaturally appearing in the cytosolic fraction of the cell in contrast to the nucleus) and damaged DNA (e.g., through mutations occurring in cancer development) is used by the immune system to identify infected or otherwise diseased cells while genomic and mitochondrial DNA present in healthy cells is ignored by DNA recognition pathways. In diseased cells, cytosolic DNA sensor proteins have been demonstrated to be involved in the detection of DNA occurring unnaturally in the cytosol of the cell. Detection of such DNA by different nucleic acid sensors translates into similar responses leading to nuclear factor kappa-B (NF-kB) and interferon type I (IFN type I) signalling followed by the activation of innate immune system components. While the recognition of viral DNA is known to induce an IFN type I response, evidence that sensing of DNA damage can initiate immune responses has only recently been accumulating.

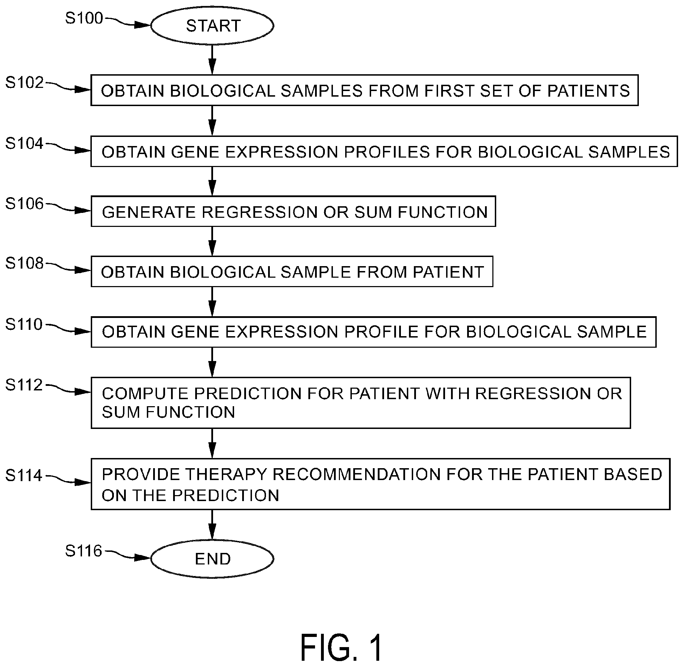

TLR9 (Toll-like receptor 9) located in the endosomes was one of the first DNA sensors molecules identified to be involved in the immune recognition of DNA by signalling downstream via the adaptor protein myeloid differentiation primary-response protein88 (MYD88). This interaction in turn activates mitogen-activated protein kinases (MAPKs) and NF-kB. TLR9 also induces the generation of type I interferons through the activation of IRF7 via IkB Kinase alpha (IKKalpha) in plasmacytoid dendritic cells (pDCs). Various other DNA immune receptors including IFI16 (IFN-gamma-inducible protein 16), cGAS (cyclic DMP-AMP synthase, DDX41 (DEAD-box helicase 41), as well as ZBP1 (Z-DNA-binding protein 1) interact with STING (stimulator of IFN genes), which activates the IKK complex and IRF3 through TBK1 (TANK binding kinase 1). ZBP1 also activates NF-kB via recruitment of RIP1 and RIP3 (receptor-interacting protein 1 and 3, respectively). While the helicase DHX36 (DEAH-box helicase 36) interacts in a complex with TRID to induce NF-kB and IRF-3/7 the DHX9 helicase stimulates MYD88-dependent signalling in plasmacytoid dendritic cells. The DNA sensor LRRFIP1 (leucine-rich repeat flightless-interacting protein) complexes with beta-catenin to activate the transcription of IRF3 whereas AIM2 (absent in melanoma 2) recruits the adaptor protein ASC (apoptosis speck-like protein) to induce a caspase-1-activating inflammasome complex leading to the secretion of interleukin-1beta (IL-1beta) and IL-18 (see Fig. 1 of Gasser S. et al., 2017, ibid, which provides a schematic overview of DNA damage and DNA sensor pathways leading to the production of inflammatory cytokines and the expression of ligands for activating innate immune receptors. Members of the non-homologous end joining pathway (orange), homologous recombination (red), inflammasome (dark green), NF-kB and interferon responses (light green) are shown).

The factors and mechanisms responsible for activating the DNA sensor pathways in cancer are currently not well elucidated. It will be important to identify the intratumoral DNA species, sensors and pathways implicated in the expression of IFNs in different cancer types at all stages of the disease. In addition to therapeutic targets in cancer, such factors may also have prognostic and predictive value. Novel DNA sensor pathway agonists and antagonists are currently being developed and tested in preclinical trials. Such compounds will be useful in characterizing the role of DNA sensor pathways in the pathogenesis of cancer, autoimmunity and potentially other diseases.

The identified immune defense response genes AIM2, APOBEC3A, CIAO1, DDX58, DHX9, IFI16, IFIH1, IFIT1, IFIT3, LRRFIP1, MYD88, OAS1, TLR8, and ZBP1 were identified as follows: A group of 538 prostate cancer patients were treated with RP and the prostate cancer tissue was stored together with clinical (e.g., pathological Gleason grade group (pGGG), pathology state (pT stage)) as well as relevant outcome parameters (e.g., biochemical recurrence (BCR), metastatic recurrence, prostate cancer specific death (PCa death), salvage radiation treatment (SRT), salvage androgen deprivation treatment (SADT), chemotherapy (CTX)). For each of these patients, a PDE4D7 score was calculated and categorized into four PDE4D7 score classes as described in Alves de Inda M. et al., "Validation of Cyclic Adenosine Monophosphate Phosphodiesterase-4D7 for its Independent Contribution to Risk Stratification in a Prostate Cancer Patient Cohort with Longitudinal Biological Outcomes", Eur Urol Focus, Vol. 4, No. 3, pages 376-384, 2018. PDE4D7 score class 1 represents patient samples with lowest expression levels of PDE4D7, whereas PDE4D7 score class 4 represents patient samples with highest levels of PDE4D7 expression. RNASeq expression data (TPM - Transcripts Per Million) of the 538 prostate cancer subjects was then investigated for differential gene expression between the PDE4D7 score classes 1 and 4. In particular, it was determined for around 20,000 protein coding transcripts whether the mean expression level of the PDE4D7 score class 1 patients was more than twice as high as the mean expression level of the PDE4D7 score class 4 patients. This analysis resulted in 637 genes with a ratio PDE4D7 score class 1 / PDE4D7 score class 4 of > 2 with a minimum mean expression of 1 TPM in each of the four PDE4D7 score classes. These 637 genes were then further subjected to molecular pathway analysis (www.david.ncifcrf.gov), which resulted in a range of enriched annotation clusters. The annotation cluster #2 demonstrated enrichment (enrichment score: 10.8) in 30 genes with a function in defense response to viruses, negative regulation of viral genome replication as well as type I interferon signaling. A further heat map analysis confirmed that these immune defense response genes were generally higher expressed in samples from patients in PDE4D7 score class 1 than from patients in PDE4D7 score class 4. The class of genes with a function in defense response to viruses, negative regulation of viral genome replication as well as type I interferon signaling was further enriched to 61 genes (APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1) by literature search to identify additional genes with the same molecular function. A further selection from the 61 genes was made based on the combinatorial power to separate patients who died from prostate cancer vs. those who did not, resulting of a preferred set of 14 genes. It was found that the number of events (metastases, prostate cancer specific death) was enriched in sub-cohorts with a low expression of these genes compared to the total patient cohort (#538) and a sub-cohort of 151 patients undergoing salvage RT (SRT) after post-surgical disease recurrence.

The term "AIM2" refers to the Absent in Melanoma 2 gene (Ensembl: ENSG00000163568), for example, to the sequence as defined in NCBI Reference Sequence NM_004833, specifically, to the nucleotide sequence as set forth in SEQ ID NO: 1, which corresponds to the sequence of the above indicated NCBI Reference Sequence of the AIM2 transcript, and also relates to the corresponding amino acid sequence for example as set forth in SEQ ID NO:2, which corresponds to the protein sequence defined in NCBI Protein Accession Reference Sequence NP_004824 encoding the AIM2 polypeptide.

The term "AIM2" also comprises nucleotide sequences showing a high degree of homology to AIM2, e.g., nucleic acid sequences being at least 75%, 80%, 85%, 90%, 91%, 92%, 93%, 94%, 95%, 96%, 97%, 98% or 99% identical to the sequence as set forth in SEQ ID NO:1 or amino acid sequences being at least 75%, 80%, 85%, 90%, 91%, 92%, 93%, 94%, 95%, 96%, 97%, 98% or 99% identical to the sequence as set forth in SEQ ID NO:2 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:2 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 1.

The term "APOBEC3A" refers to the Apolipoprotein B mRNA Editing Enzyme Catalytic Subunit 3A gene (Ensembl: ENSG00000128383), for example, to the sequence as defined in NCBI Reference Sequence NM_145699, specifically, to the nucleotide sequence as set forth in SEQ ID NO:3, which corresponds to the sequence of the above indicated NCBI Reference Sequence of the APOBEC3A transcript, and also relates to the corresponding amino acid sequence for example as set forth in SEQ ID NO:4, which corresponds to the protein sequence defined in NCBI Protein Accession Reference Sequence NP_663745 encoding the APOBEC3A polypeptide.

The term "APOBEC3A" also comprises nucleotide sequences showing a high degree of homology to APOBEC3A, e.g., nucleic acid sequences being at least 75%, 80%, 85%, 90%, 91%, 92%, 93%, 94%, 95%, 96%, 97%, 98% or 99% identical to the sequence as set forth in SEQ ID NO:3 or amino acid sequences being at least 75%, 80%, 85%, 90%, 91%, 92%, 93%, 94%, 95%, 96%, 97%, 98% or 99% identical to the sequence as set forth in SEQ ID NO:4 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:4 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:3.

The term "CIAO1" refers to the Cytosolic Iron-Sulfur Assembly Component 1 gene (Ensembl: ENSG00000144021), for example, to the sequence as defined in NCBI Reference Sequence NM_004804, specifically, to the nucleotide sequence as set forth in SEQ ID NO:5, which corresponds to the sequence of the above indicated NCBI Reference Sequence of the CIAO1 transcript, and also relates to the corresponding amino acid sequence for example as set forth in SEQ ID NO:6, which corresponds to the protein sequence defined in NCBI Protein Accession Reference Sequence NP_663745 encoding the CIAO1 polypeptide.

The term "CIAO1" also comprises nucleotide sequences showing a high degree of homology to CIAO1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:5 or amino acid sequences being at least 75%, 80%, 85%, 90%, 91%, 92%, 93%, 94%, 95%, 96%, 97%, 98% or 99% identical to the sequence as set forth in SEQ ID NO:6 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:6 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:5.

The term "DDX58" refers to the DExD/H-box Helicase 58 gene (Ensembl: ENSG00000107201), for example, to the sequence as defined in NCBI Reference Sequence NM_014314, specifically, to the nucleotide sequence as set forth in SEQ ID NO:7, which corresponds to the sequence of the above indicated NCBI Reference Sequence of the DDX58 transcript, and also relates to the corresponding amino acid sequence for example as set forth in SEQ ID NO:8, which corresponds to the protein sequence defined in NCBI Protein Accession Reference Sequence NP_055129 encoding the DDX58 polypeptide.

The term "DDX58" also comprises nucleotide sequences showing a high degree of homology to DDX58, e.g., nucleic acid sequences being at least 75%, 80%, 85%, 90%, 91%, 92%, 93%, 94%, 95%, 96%, 97%, 98% or 99% identical to the sequence as set forth in SEQ ID NO:7 or amino acid sequences being at least 75%, 80%, 85%, 90%, 91%, 92%, 93%, 94%, 95%, 96%, 97%, 98% or 99% identical to the sequence as set forth in SEQ ID NO:8 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:8 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:7.

The term "DHX9" refers to the DExD/H-box Helicase 9 gene (Ensembl: ENSG00000135829), for example, to the sequence as defined in NCBI Reference Sequence NM_001357, specifically, to the nucleotide sequence as set forth in SEQ ID NO:9, which corresponds to the sequence of the above indicated NCBI Reference Sequence of the DHX9 transcript, and also relates to the corresponding amino acid sequence for example as set forth in SEQ ID NO: 10, which corresponds to the protein sequence defined in NCBI Protein Accession Reference Sequence NP_001348 encoding the DHX9 polypeptide.

The term "DHX9" also comprises nucleotide sequences showing a high degree of homology to DHX9, e.g., nucleic acid sequences being at least 75%, 80%, 85%, 90%, 91%, 92%, 93%, 94%, 95%, 96%, 97%, 98% or 99% identical to the sequence as set forth in SEQ ID NO:9 or amino acid sequences being at least 75%, 80%, 85%, 90%, 91%, 92%, 93%, 94%, 95%, 96%, 97%, 98% or 99% identical to the sequence as set forth in SEQ ID NO:10 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 10 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:9.

The term "IFI16" refers to the Interferon Gamma Inducible Protein 16 gene (Ensembl: ENSG00000163565), for example, to the sequence as defined in NCBI Reference Sequence NM_005531, specifically, to the nucleotide sequence as set forth in SEQ ID NO:11, which corresponds to the sequence of the above indicated NCBI Reference Sequence of the IFI16 transcript, and also relates to the corresponding amino acid sequence for example as set forth in SEQ ID NO:12, which corresponds to the protein sequence defined in NCBI Protein Accession Reference Sequence NP_005522 encoding the IFI16 polypeptide.

The term "IFI16" also comprises nucleotide sequences showing a high degree of homology to IFI16, e.g., nucleic acid sequences being at least 75%, 80%, 85%, 90%, 91%, 92%, 93%, 94%, 95%, 96%, 97%, 98% or 99% identical to the sequence as set forth in SEQ ID NO:11 or amino acid sequences being at least 75%, 80%, 85%, 90%, 91%, 92%, 93%, 94%, 95%, 96%, 97%, 98% or 99% identical to the sequence as set forth in SEQ ID NO: 12 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:12 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:11.

The term "IFIH1" refers to the Interferon Induced With Helicase C Domain 1 gene (Ensembl: ENSG00000115267), for example, to the sequence as defined in NCBI Reference Sequence NM_022168, specifically, to the nucleotide sequence as set forth in SEQ ID NO: 13, which corresponds to the sequence of the above indicated NCBI Reference Sequence of the IFIH1 transcript, and also relates to the corresponding amino acid sequence for example as set forth in SEQ ID NO: 14, which corresponds to the protein sequence defined in NCBI Protein Accession Reference Sequence NP_071451 encoding the IFIH1 polypeptide.

The term "IFIH1" also comprises nucleotide sequences showing a high degree of homology to IFIH1, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 13 or amino acid sequences being at least 75%, 80%, 85%, 90%, 91%, 92%, 93%, 94%, 95%, 96%, 97%, 98% or 99% identical to the sequence as set forth in SEQ ID NO: 14 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO: 14 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO: 13.

The term "IFIT1" refers to the Interferon Induced Protein With Tetratricopeptide Repeats 1 gene (Ensembl: ENSG00000185745), for example, to the sequence as defined in NCBI Reference Sequence NM_001270929 or in NCBI Reference Sequence NM_001548.5, specifically, to the nucleotide sequence as set forth in SEQ ID NO: 15 or in SEQ ID NO: 16, which correspond to the sequences of the above indicated NCBI Reference Sequences of the IFIT1 transcript, and also relates to the corresponding amino acid sequence for example as set forth in SEQ ID NO: 17 or in SEQ ID NO: 18, which correspond to the protein sequences defined in NCBI Protein Accession Reference Sequence NP_001257858 and in NCBI Protein Accession Reference Sequence NP_001539 encoding the IFIT1 polypeptide.

The term "IFIT1" also comprises nucleotide sequences showing a high degree of homology to IFIT1, e.g., nucleic acid sequences being at least 75%, 80%, 85%, 90%, 91%, 92%, 93%, 94%, 95%, 96%, 97%, 98% or 99% identical to the sequence as set forth in SEQ ID NO: 15 or in SEQ ID NO: 16 or amino acid sequences being at least 75%, 80%, 85%, 90%, 91%, 92%, 93%, 94%, 95%, 96%, 97%, 98% or 99% identical to the sequence as set forth in SEQ ID NO: 17 or in SEQ ID NO:18 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:17 or SEQ ID NO:18 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:15 or in SEQ ID NO:16.

The term "IFIT3" refers to the Interferon Induced Protein With Tetratricopeptide Repeats 3 gene (Ensembl: ENSG00000119917), for example, to the sequence as defined in NCBI Reference Sequence NM_001031683, specifically, to the nucleotide sequence as set forth in SEQ ID NO:19, which corresponds to the sequence of the above indicated NCBI Reference Sequence of the IFIT3 transcript, and also relates to the corresponding amino acid sequence for example as set forth in SEQ ID NO:20, which corresponds to the protein sequence defined in NCBI Protein Accession Reference Sequence NP_001026853 encoding the IFIT3 polypeptide.

The term "IFIT3" also comprises nucleotide sequences showing a high degree of homology to IFIT3, e.g., nucleic acid sequences being at least 75%, 80%, 85%, 90%, 91%, 92%, 93%, 94%, 95%, 96%, 97%, 98% or 99% identical to the sequence as set forth in SEQ ID NO:19 or amino acid sequences being at least 75%, 80%, 85%, 90%, 91%, 92%, 93%, 94%, 95%, 96%, 97%, 98% or 99% identical to the sequence as set forth in SEQ ID NO:20 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:20 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:19.

The term "LRRFIP1" refers to the LRR Binding FLII Interacting Protein 1 gene (Ensembl: ENSG00000124831), for example, to the sequence as defined in NCBI Reference Sequence NM_004735 or in NCBI Reference Sequence NM_001137550 or in NCBI Reference Sequence NM_001137553 or in NCBI Reference Sequence NM_001137552, specifically, to the nucleotide sequence as set forth in SEQ ID NO:21 or in SEQ ID NO:22 or in SEQ ID NO:23 or in SEQ ID NO:24, which correspond to the sequences of the above indicated NCBI Reference Sequences of the LRRFIP1 transcript, and also relates to the corresponding amino acid sequence for example as set forth in SEQ ID NO:25 or in SEQ ID NO:26 or in SEQ ID NO:27 or in SEQ ID NO:28, which correspond to the protein sequences defined in NCBI Protein Accession Reference Sequence NP_004726 and in NCBI Protein Accession Reference Sequence NP_001131022 and in NCBI Protein Accession Reference Sequence NP_001131025 and in NCBI Protein Accession Reference Sequence NP_001131024 encoding the LRRFIP1 polypeptide.

The term "LRRFIP1" also comprises nucleotide sequences showing a high degree of homology to LRRFIP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or in SEQ ID NO:23 or in SEQ ID NO:24 or amino acid sequences being at least 75%, 80%, 85%, 90%, 91%, 92%, 93%, 94%, 95%, 96%, 97%, 98% or 99% identical to the sequence as set forth in SEQ ID NO:25 or in SEQ ID NO:26 or in SEQ ID NO:27 or in SEQ ID NO:28 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:25 or in SEQ ID NO:26 or in SEQ ID NO:27 or in SEQ ID NO:28 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:21 or in SEQ ID NO:22 or in SEQ ID NO:23 or in SEQ ID NO:24.

The term "MYD88" refers to the MYD88 Innate Immune Signal Transduction Adaptor gene (Ensembl: ENSG00000172936), for example, to the sequence as defined in NCBI Reference Sequence NM_001172567 or in NCBI Reference Sequence NM_001172568 or in NCBI Reference Sequence NM_001172569 or in NCBI Reference Sequence NM_001172566 or in NCBI Reference Sequence NM_002468, specifically, to the nucleotide sequences as set forth in SEQ ID NO:29 or in SEQ ID NO:30 or in SEQ ID NO:31 or in SEQ ID NO:32 or in SEQ ID NO:33, which correspond to the sequences of the above indicated NCBI Reference Sequences of the MYD88 transcript, and also relates to the corresponding amino acid sequence for example as set forth in SEQ ID NO:34 or in SEQ ID NO:35 or in SEQ ID NO:36 or in SEQ ID NO:37 or in SEQ ID NO:38, which correspond to the protein sequences defined in NCBI Protein Accession Reference Sequence NP_001166038 and in NCBI Protein Accession Reference Sequence NP_001166039 and in NCBI Protein Accession Reference Sequence NP_001166040 and in NCBI Protein Accession Reference Sequence NP_001166037 and in NCBI Protein Accession Reference Sequence NP_002459 encoding the MYD88 polypeptide.

The term "MYD88" also comprises nucleotide sequences showing a high degree of homology to MYD88, e.g., nucleic acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or in SEQ ID NO:31 or in SEQ ID NO:32 or in SEQ ID NO:33 or amino acid sequences being at least 75%, 80%, 85%, 90%, 91%, 92%, 93%, 94%, 95%, 96%, 97%, 98% or 99% identical to the sequence as set forth in SEQ ID NO:34 or in SEQ ID NO:35 or in SEQ ID NO:36 or in SEQ ID NO:37 or in SEQ ID NO:38 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:34 or in SEQ ID NO:35 or in SEQ ID NO:36 or in SEQ ID NO:37 or in SEQ ID NO:38 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:29 or in SEQ ID NO:30 or in SEQ ID NO:31 or in SEQ ID NO:32 or in SEQ ID NO:33.

The term "OAS1" refers to the 2'-5'-Oligoadenylate Synthetase 1 gene (Ensembl: ENSG00000089127), for example, to the sequence as defined in NCBI Reference Sequence NM_001320151 or in NCBI Reference Sequence NM_002534 or in NCBI Reference Sequence NM_001032409 or in NCBI Reference Sequence NM_016816, specifically, to the nucleotide sequences as set forth in SEQ ID NO:39 or in SEQ ID NO:40 or in SEQ ID NO:41 or in SEQ ID NO:42, which correspond to the sequences of the above indicated NCBI Reference Sequences of the OAS1 transcript, and also relates to the corresponding amino acid sequences for example as set forth in SEQ ID NO:43 or in SEQ ID NO:44 or in SEQ ID NO:45 or in SEQ ID NO:46, which correspond to the protein sequences defined in NCBI Protein Accession Reference Sequence NP_001307080 and in NCBI Protein Accession Reference Sequence NP_002525 and in NCBI Protein Accession Reference Sequence NP_001027581 and in NCBI Protein Accession Reference Sequence NP_058132 encoding the OAS1 polypeptide.

The term "OAS1" also comprises nucleotide sequences showing a high degree of homology to OAS1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:39 or in SEQ ID NO:40 or in SEQ ID NO:41 or in SEQ ID NO:42 or amino acid sequences being at least 75%, 80%, 85%, 90%, 91%, 92%, 93%, 94%, 95%, 96%, 97%, 98% or 99% identical to the sequence as set forth in SEQ ID NO:43 or in SEQ ID NO:44 or in SEQ ID NO:45 or in SEQ ID NO:46 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:43 or in SEQ ID NO:44 or in SEQ ID NO:45 or in SEQ ID NO:46 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:39 or in SEQ ID NO:40 or in SEQ ID NO:41 or in SEQ ID NO:42.

The term "TLR8" refers to the Toll Like Receptor 8 gene (Ensembl: ENSG00000101916), for example, to the sequence as defined in NCBI Reference Sequence NM_138636 or in NCBI Reference Sequence NM_016610, specifically, to the nucleotide sequences as set forth in SEQ ID NO:47 or in SEQ ID NO:48, which correspond to the sequences of the above indicated NCBI Reference Sequences of the TLR8 transcript, and also relates to the corresponding amino acid sequences for example as set forth in SEQ ID NO:49 or in SEQ ID NO:50, which corresponds to the protein sequences defined in NCBI Protein Accession Reference Sequence NP_619542 and in NCBI Protein Accession Reference Sequence NP_057694 encoding the TLR8 polypeptide.

The term "TLR8" also comprises nucleotide sequences showing a high degree of homology to TLR8, e.g., nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48 or amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:49 or in SEQ ID NO:50 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:47 or in SEQ ID NO:48.

The term "ZBP1" refers to the Z-DNA Binding Protein 1 gene (Ensembl: ENSG00000124256), for example, to the sequence as defined in NCBI Reference Sequence NM_030776 or in NCBI Reference Sequence NM_001160418 or in NCBI Reference Sequence NM_001160419, specifically, to the nucleotide sequence as set forth in SEQ ID NO:51 or in SEQ ID NO:52 or in SEQ ID NO:53, which corresponds to the sequence of the above indicated NCBI Reference Sequence of the ZBP1 transcript, and also relates to the corresponding amino acid sequence for example as set forth in SEQ ID NO:54 or in SEQ ID NO:55 or in SEQ ID NO:56, which corresponds to the protein sequences defined in NCBI Protein Accession Reference Sequence NP_110403 and in NCBI Protein Accession Reference Sequence NP_001153890 and in NCBI Protein Accession Reference Sequence NP_001153891 encoding the ZBP1 polypeptide.

The term "ZBP1" also comprises nucleotide sequences showing a high degree of homology to ZBP1, e.g., nucleic acid sequences being at least 75%, 80%, 85%, 90%, 91%, 92%, 93%, 94%, 95%, 96%, 97%, 98% or 99% identical to the sequence as set forth in SEQ ID NO:51 or in SEQ ID NO:52 or in SEQ ID NO:53 or amino acid sequences being at least 75%, 80%, 85%, 90%, 91%, 92%, 93%, 94%, 95%, 96%, 97%, 98% or 99% identical to the sequence as set forth in SEQ ID NO:54 or in SEQ ID NO:55 or in SEQ ID NO:56 or nucleic acid sequences encoding amino acid sequences being at least 75%, 80%, 85%, 90%, 91%, 92%, 93%, 94%, 95%, 96%, 97%, 98% or 99% identical to the sequence as set forth in SEQ ID NO:54 or in SEQ ID NO:55 or in SEQ ID NO:56 or amino acid sequences being encoded by nucleic acid sequences being at least 75%, 80%, 85%, 90%, 91%, 92%, 93%, 94%, 95%, 96%, 97%, 98% or 99% identical to the sequence as set forth in SEQ ID NO:51 or in SEQ ID NO:52 or in SEQ ID NO:53.

The term "biological sample" or "sample obtained from a subject" refers to any biological material obtained via suitable methods known to the person skilled in the art from a subject, e.g., a prostate cancer patient. The biological sample used may be collected in a clinically acceptable manner, e.g., in a way that nucleic acids (in particular RNA) or proteins are preserved.

The biological sample(s) may include body tissue and/or a fluid, such as, but not limited to, blood, sweat, saliva, and urine. Furthermore, the biological sample may contain a cell extract derived from or a cell population including an epithelial cell, such as a cancerous epithelial cell or an epithelial cell derived from tissue suspected to be cancerous. The biological sample may contain a cell population derived from a glandular tissue, e.g., the sample may be derived from the prostate of a male subject. Additionally, cells may be purified from obtained body tissues and fluids if necessary, and then used as the biological sample. In some realizations, the sample may be a tissue sample, a urine sample, a urine sediment sample, a blood sample, a saliva sample, a semen sample, a sample including circulating tumour cells, extracellular vesicles, a sample containing prostate secreted exosomes, or cell lines or cancer cell line.

In one particular realization, biopsy or resections samples may be obtained and/or used. Such samples may include cells or cell lysates.

It is also conceivable that the content of a biological sample is submitted to an enrichment step. For instance, a sample may be contacted with ligands specific for the cell membrane or organelles of certain cell types, e.g., prostate cells, functionalized for example with magnetic particles. The material concentrated by the magnetic particles may subsequently be used for detection and analysis steps as described herein above or below.

Furthermore, cells, e.g., tumour cells, may be enriched via filtration processes of fluid or liquid samples, e.g., blood, urine, etc. Such filtration processes may also be combined with enrichment steps based on ligand specific interactions as described herein above.

The term "prostate cancer" refers to a cancer of the prostate gland in the male reproductive system, which occurs when cells of the prostate mutate and begin to multiply out of control. Typically, prostate cancer is linked to an elevated level of prostate-specific antigen (PSA). In one embodiment of the present invention the term "prostate cancer" relates to a cancer showing PSA levels above 3.0. In another embodiment the term relates to cancer showing PSA levels above 2.0. The term "PSA level" refers to the concentration of PSA in the blood in ng/ml.

The term "non-progressive prostate cancer state" means that a sample of an individual does not show parameter values indicating "biochemical recurrence" and/or "clinical recurrence" and/or "metastases" and/or "castration-resistant disease" and/or "prostate cancer or disease specific death".

The term "progressive prostate cancer state" means that a sample of an individual shows parameter values indicating "biochemical recurrence" and/or "clinical recurrence" and/or "metastases" and/or "castration-resistant disease" and/or "prostate cancer or disease specific death".

The term "biochemical recurrence" generally refers to recurrent biological values of increased PSA indicating the presence of prostate cancer cells in a sample. However, it is also possible to use other markers that can be used in the detection of the presence or that rise suspicion of such presence.

The term "clinical recurrence" refers to the presence of clinical signs indicating the presence of tumour cells as measured, for example using in vivo imaging.

The term "metastases" refers to the presence of metastatic disease in organs other than the prostate.

The term "castration-resistant disease" refers to the presence of hormone-insensitive prostate cancer; i.e., a cancer in the prostate that does not any longer respond to androgen deprivation therapy (ADT).

The term "prostate cancer specific death or disease specific death" refers to death of a patient from his prostate cancer.

It is preferred that the five or more immune defense response genes comprise six or more of the immune defense response genes.

It is further preferred that the five or more immune defense response genes comprise nine or more of the immune defense response genes.

It is yet further preferred that the five or more immune defense response genes comprise twelve or more.

It is preferred that the determining of the prediction of the radiotherapy response comprises combining the gene expression profiles for five or more, for example, 5, 6, 7, 8, 9, 10, 11, 12, 13 or all, of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, with a regression function that had been derived from a population of prostate cancer subjects.

Cox proportional-hazards regression allows analyzing the effect of several risk factors on time to a tested event like survival. Thereby, the risk factors maybe dichotomous or discrete variables like a risk score or a clinical stage but may also be a continuous variable like a biomarker measurement or gene expression values. The probability of the endpoint (e.g., death or disease recurrence) is called the hazard. Next to the information on whether or not the tested endpoint was reached by e.g. subject in a patient cohort (e.g., patient did die or not) also the time to the endpoint is considered in the regression analysis. The hazard is modeled as: $H(t)=H_0(t) \cdot \exp(w_1 \cdot V_1 + w_2 \cdot V_2 + w_3 \cdot V_3 + ...)$, where $V_1$, $V_2$, $V_3$ ... are predictor variables and $H_0(t)$ is the baseline hazard while $H(t)$ is the hazard at any time t. The hazard ratio (or the risk to reach the event) is represented by $\text{Ln}[H(t)/ H_0(t)] = w_1 \cdot V_1 + w_2 \cdot V_2 + w_3 \cdot V_3 + ...$, where the coefficients or weights $w_1$, $w_2$, $w_3$ ... are estimated by the Cox regression analysis and can be interpreted in a similar manner as for logistic regression analysis.

In one particular realization, the prediction of the radiotherapy response is determined as follows:

$$(w_1 \cdot AIM2) + (w_2 \cdot APOBEC3A) + (w_3 \cdot CIAO1) + (w_4 \cdot DDX58) + (w_5 \cdot DHX9) + (w_6 \cdot IFI16) + (w_7 \cdot IFIH1) + (w_8 \cdot IFIT1) + (w_9 \cdot IFIT3) + (w_{10} \cdot LRRFIP1) + (w_{11} \cdot MYD88) + (w_{12} \cdot OAS1) + (w_{13} \cdot TLR8) + (w_{14} \cdot ZBP1) \tag{1}$$

where $w_1$ to $w_{14}$ are weights and AIM2, APOBEC3A, CIAO1, DDX58, DHX9, IFI16, IFIH1, IFIT1, IFIT3, LRRFIP1, MYD88, OAS1, TLR8, and ZBP1 are the expression levels of the immune defense response genes.

In one example, $w_1$ may be about -0.5 to 0.5, such as -0.02116, $w_2$ may be about -0.5 to -0.5, such as -0.01195, $w_3$ may be about -0.5 to 0.5, such as -0.02092, $w_4$ may be about -0.5 to 0.5, such as -0.009288, $w_5$ may be about -0.5 to 0.5, such as 0.002662, $w_6$ may be about -0.5 to 0.5, such as -0.01924, $w_7$ may be about -0.5 to 0.5, such as 0.007922, $w_8$ may be about -0.5 to 0.5, such as -0.06404, $w_9$ may be about -0.5 to 0.5, such as 0.01869, $w_{10}$ may be about -0.5 to 0.5, such as -0.005425, $w_{11}$ may be about -0.5 to 0.5, such as 0.002878, $w_{12}$ may be about -0.5 to 0.5, such as 0.001348, $w_{13}$ may be about -0.5 to 0.5, such as - 0.04764, and $w_{14}$ may be about -0.5 to 0.5, such as -0.01494.

Alternatively, it is preferred that the determining of the prediction of the radiotherapy response comprises combining the gene expression profiles for five or more, for example, 5, 6, 7, 8, 9, 10, 11, 12, 13 or all, of the immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, with a sum function that sums discretized gene expression profiles of the five or more immune defense response genes, wherein the gene expression profiles are discretized using respective cut-offs that had been derived from a population of prostate cancer subjects.

In one particular realization, the prediction of the radiotherapy response is determined as follows:

```
For each immune defense response gene i
   if( EL(i) < cut-off(i) )
      BEL(i) = 0
    else
      BEL(i) = 1 (2)
if ( sum( BEL(i) < thresh )
   IDR_score = 0
else
   IDR_score = 1
```

where EL(i) and cut-off(i) are the expression level and the cut-off of the i-th immune defense response gene, respectively, BEL(i) is the binarized expression level of the i-th immune defense response gene, IDR_score is the immune defense response score, and thresh is a threshold. Instead of using a binarization as in Eq. (2), other discretizations may be used, e.g., a gene expression profile may be mapped to one of three or four values using two or three cut-offs.

In one example, the cut-off for AIM2 may be about 30 to 40, such as 33.44, the cut-off for APOBEC3A may be about 5 to 15, such as 8.55, the cut-off for CIAO1 may be about 60 to 70, such as 64.49, the cut-off for DDX58 may be about 40 to 50, such as 45.87, the cut-off for DHX9 may be about 200 to 250, such as 230.03, the cut-off for IFI16 may be about 50 to 60, such as 53.56, the cut-off for IFIH1 may be about 90 to 110, such as 99.63, the cut-off for IFIT1 may be about 10 to 20, such as 14.57, the cut-off for IFIT3 may be about 5 to 15, such as 11.75, the cut-off for LRRFIP1 may be about 260 to 310, such as 283.82, the cut-off for MYD88 may be about 20 to 30, such as 23.03, the cut-off for OAS1 may be about 30 to 40, such as 33.93, the cut-off for TLR8 may be about 5 to 15, such as 9.89, the cut-off for ZBP1 may be about 15 to 25, such as 20.16, and thresh may be about 0 to 5, such as 1.

The prediction of the radiotherapy response may also be classified or categorized into one of at least two risk groups, based on the value of the prediction of the radiotherapy response. For example, there may be two risk groups, or three risk groups, or four risk groups, or more than four predefined risk groups. Each risk group covers a respective range of (non-overlapping) values of the prediction of the radiotherapy response. For example, a risk group may indicate a probability of occurrence of a specific clinical event from 0 to <0.1 or from 0.1 to <0.25 or from 0.25 to <0.5 or from 0.5 to 1.0 or the like.

It is preferred that the biological sample is obtained from the subject before the start of the radiotherapy. The gene expression profiles may be determined in the form of mRNA or protein in tissue of prostate cancer. Alternatively, if the immune defense response genes are present in a soluble form, the gene expression profiles may be determined in blood.

It is further preferred that the radiotherapy is radical radiotherapy or salvage radiotherapy.

It is preferred that the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy. The degree to which the prediction is negative may determine the degree to which the recommended therapy deviates from the standard form of radiotherapy.

In a further aspect of the present invention, an apparatus for predicting a response of a prostate cancer subject to radiotherapy is presented, comprising:

- an input adapted to receive data indicative of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
- a processor adapted to determine the prediction of the radiotherapy response based on the gene expression profiles for the five or more immune defense response genes, and
- optionally, a providing unit adapted to provide the prediction or a therapy recommendation based on the prediction to a medical caregiver or the subject.

In a further aspect of the present invention, a computer program product is presented comprising instructions which, when the program is executed by a computer, cause the computer to carry out a method comprising:

- receiving data indicative of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
- determining the prediction of the radiotherapy response based on the gene expression profiles for the five or more immune defense response genes, and
- optionally, providing the prediction or a therapy recommendation based on the prediction to a medical caregiver or the subject.

In a further aspect of the present invention, a diagnostic kit is presented, comprising:

- at least five primers and/or probes for determining the gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and

- an apparatus as defined in claim 11 or a computer program product as defined in claim 12.

In a further aspect of the present invention, a use of the kit as defined in claim 13 is presented.

It is preferred that the use as defined in claim 14 is in a method of predicting a response of a prostate cancer subject to radiotherapy.

In a further aspect of the present invention, a method is presented, comprising:

- receiving a biological sample obtained from a prostate cancer subject,
- using the kit as defined in claim 12 to determine a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in the biological sample obtained from the subject.

In a further aspect of the present invention, a use of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a method of predicting a response of a prostate cancer subject to radiotherapy is presented, comprising:

- determining, preferably by a processor, the prediction of the radiotherapy response based on the gene expression profiles for the five or more immune defense response genes, and
- optionally, providing the prediction or a therapy recommendation based on the prediction to a medical caregiver or the subject.

It shall be understood that the method of claim 1, the apparatus of claim 10, the computer program product of claim 11, the diagnostic kit of claim 12, the use of the diagnostic kit of claim 13, the method of claim 15, and the use of the gene expression profiles of claim 16 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

In the following drawings:

Fig. 1 shows schematically and exemplarily a flowchart of an embodiment of a method of predicting a response of a prostate cancer subject to radiotherapy.

Figs. 2 and 3 show a Kaplan-Meier curve analysis of the immune defense response model (IDR_model). The clinical endpoint that was tested was the time to prostate cancer specific death (PCa Death) after the start of the salvage radiation therapy (SRT) due to post-surgical disease recurrence.

Fig. 4 shows a Kaplan-Meier curve analysis of the immune defense response score (IDR_score) for men with post-surgical recurrent prostate cancer undergoing salvage radiation therapy (SRT). The clinical endpoint that was tested was prostate cancer specific death (PCa Death) after the start of SRT.

Fig. 5 shows a Kaplan-Meier curve analysis of the CARPA-S score categories for men with post-surgical recurrent prostate cancer undergoing salvage radiation therapy (SRT). The clinical endpoint that was tested was prostate cancer specific death (PCa Death) after the start of SRT.

Fig. 6 shows a Kaplan-Meier curve analysis of the EAU BCR risk groups (EAU_BCR_Risk) for men with post-surgical recurrent prostate cancer undergoing salvage radiation therapy (SRT). The clinical endpoint that was tested was prostate cancer specific death (PCa Death) after the start of SRT.

Fig. 7 shows a Kaplan-Meier curve analysis of the immune defense response score (IDR_score) for men with post-surgical recurrent prostate cancer undergoing salvage androgen deprivation therapy (SADT). The clinical endpoint that was tested was prostate cancer specific death (PCa Death) after the start of SADT.

Fig. 8 shows a Kaplan-Meier curve analysis of the CARPA-S score categories for men with post-surgical recurrent prostate cancer undergoing salvage androgen deprivation therapy (SADT). The clinical endpoint that was tested was prostate cancer specific death (PCa Death) after the start of SADT.

Fig. 9 shows a Kaplan-Meier curve analysis of the EAU BCR risk groups (EAU_BCR_Risk) for men with post-surgical recurrent prostate cancer undergoing salvage radiation treatment (SRT). The clinical endpoint that was tested was prostate cancer specific death (PCa Death) after the start of SRT.

Fig. 10 shows a Kaplan-Meier curve analysis of the immune defense response score (IDR_score) for men with post-surgical recurrent prostate cancer undergoing cytotoxic therapy (CTX). The clinical endpoint that was tested was prostate cancer specific death (PCa Death) after the start of CTX.

Fig. 11 shows a Kaplan-Meier curve analysis of the CARPA-S score categories for men with post-surgical recurrent prostate cancer undergoing cytotoxic therapy (CTX). The clinical endpoint that was tested was prostate cancer specific death (PCa Death) after the start of CTX.

Fig. 12 shows a Kaplan-Meier curve analysis of the EAU BCR risk groups (EAU_BCR_Risk) for men with post-surgical recurrent prostate cancer undergoing cytotoxic therapy (CTX). The clinical endpoint that was tested was prostate cancer specific death (PCa Death) after the start of CTX.

Fig. 13 shows a Kaplan-Meier curve analysis of the immune defense response score (IDR_score) for men with post-surgical recurrent prostate cancer. The clinical endpoint that was tested was metastases-free survival after primary treatment.

Fig. 14 shows a Kaplan-Meier curve analysis of the CARPA-S score categories for men with post-surgical recurrent prostate cancer. The clinical endpoint that was tested was metastases-free survival after primary treatment.

Fig. 15 shows a Kaplan-Meier curve analysis of the immune defense response score (IDR_score) for men with post-surgical recurrent prostate cancer. The clinical endpoint that was tested was metastases-free survival after post-surgical biochemical recurrence (BCR).

Fig. 16 shows a Kaplan-Meier curve analysis of the CARPA-S score categories for men with post-surgical recurrent prostate cancer. The clinical endpoint that was tested was metastases-free survival after post-surgical biochemical recurrence (BCR).

Fig. 17 shows a Kaplan-Meier curve analysis of the immune defense response score (IDR_score) for men with post-surgical recurrent prostate cancer. The clinical endpoint that was tested was overall survival after primary treatment.

Fig. 18 shows a Kaplan-Meier curve analysis of the pathology Gleason grade group (pGGG) for men with post-surgical recurrent prostate cancer. The clinical endpoint that was tested was overall survival after primary treatment.

Fig. 19 shows a Kaplan-Meier curve analysis of the immune defense response score (IDR_score) for men with post-surgical recurrent prostate cancer. The tested endpoint was overall survival after post-surgical biochemical recurrence (BCR).

Fig. 20 shows a Kaplan-Meier curve analysis of the pathology Gleason grade group (pGGG) for men with post-surgical recurrent prostate cancer. The tested endpoint was overall survival after post-surgical biochemical recurrence (BCR).

Fig. 21 shows a Kaplan-Meier curve of a Immune defense response 5 gene model (IDR_5.1_model). The clinical endpoint that was tested was the time to prostate cancer specific death (PCa Death) after the start of salvage radiation therapy (SRT) due to post-surgical disease recurrence.

Fig. 22 shows a Kaplan-Meier curve of another Immune defense response 5 gene model (IDR_5.2_model). The clinical endpoint that was tested was the time to prostate cancer specific death (PCa Death) after the start of salvage radiation therapy (SRT) due to post-surgical disease recurrence.

Fig. 23 shows a Kaplan-Meier curve of another Immune defense response 5 gene model (IDR_5.3_model). The clinical endpoint that was tested was the time to prostate cancer specific death (PCa Death) after the start of salvage radiation therapy (SRT) due to post-surgical disease recurrence.

Fig. 24 shows a Kaplan-Meier curve of another Immune defense response 5 gene model (IDR_5.4_model). The clinical endpoint that was tested was the time to prostate cancer specific death (PCa Death) after the start of salvage radiation therapy (SRT) due to post-surgical disease recurrence.

Fig. 25 shows a Kaplan-Meier curve of another Immune defense response 5 gene model (IDR_5.5_model). The clinical endpoint that was tested was the time to prostate cancer specific death (PCa Death) after the start of salvage radiation therapy (SRT) due to post-surgical disease recurrence.

Fig. 26 shows a Kaplan-Meier curve of another Immune defense response 5 gene model (IDR_5.6_ model). The clinical endpoint that was tested was the time to prostate cancer specific death (PCa Death) after the start of salvage radiation therapy (SRT) due to post-surgical disease recurrence.

Fig. 27 shows a Kaplan-Meier curve of another Immune defense response 5 gene model (IDR _5.7_model). The clinical endpoint that was tested was the time to prostate cancer specific death (PCa Death) after the start of salvage radiation therapy (SRT) due to post-surgical disease recurrence.

Fig. 28 shows a Kaplan-Meier curve of another Immune defense response 5 gene model (IDR_5.8_model). The clinical endpoint that was tested was the time to prostate cancer specific death (PCa Death) after the start of salvage radiation therapy (SRT) due to post-surgical disease recurrence.

Fig. 29 shows a Kaplan-Meier curve of a Immune defense response 6 gene model (IDR_6.1_model). The clinical endpoint that was tested was the time to prostate cancer specific death (PCa Death) after the start of salvage radiation therapy (SRT) due to post-surgical disease recurrence.

Fig. 30 shows a Kaplan-Meier curve of another Immune defense response 6 gene model (IDR_6.2_model). The clinical endpoint that was tested was the time to prostate cancer specific death (PCa Death) after the start of salvage radiation therapy (SRT) due to post-surgical disease recurrence.

Fig. 31 shows a Kaplan-Meier curve of another Immune defense response 6 gene model (IDR_6.3_model). The clinical endpoint that was tested was the time to prostate cancer specific death (PCa Death) after the start of salvage radiation therapy (SRT) due to post-surgical disease recurrence.

Fig. 32 shows a Kaplan-Meier curve of another Immune defense response 6 gene model (IDR_6.4_model). The clinical endpoint that was tested was the time to prostate cancer specific death (PCa Death) after the start of salvage radiation therapy (SRT) due to post-surgical disease recurrence.

## DETAILED DESCRIPTION OF EMBODIMENTS

### Overview Of Radiotherapy Response Prediction

Fig. 1 shows schematically and exemplarily a flowchart of an embodiment of a method of predicting a response of a prostate cancer subject to radiotherapy.

The method begins at step S100.

At step S102, a biological sample is obtained from each of a first set of patients (subjects) diagnosed with prostate cancer. Preferably, monitoring prostate cancer has been performed for these prostate cancer patients over a period of time, such as at least one year, or at least two years, or about five years, after obtaining the biological sample.

At step S104, a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, is obtained for each of the biological samples obtained from the first set of patients, e.g., by performing RT-qPCR (real-time quantitative PCR) on RNA extracted from each biological sample. The exemplary gene expression profiles include an expression level (e.g., value) for each of the five or more immune defense response genes.

At step S106, a regression function for assigning a prediction of the radiotherapy response is determined based on the gene expression profiles for the five or more immune defense response genes, AIM2, APOBEC3A, CIAO1, DDX58, DHX9, IFI16, IFIH1, IFIT1, IFIT3, LRRFIP1, MYD88, OAS1, TLR8, and/or ZBP1, obtained for at least some of the biological samples obtained for the first set of patients and respective results obtained from the monitoring. In one particular realization, the regression function is determined as specified in Eq. (1) above.

At step S108, a biological sample is obtained from a patient (subject or individual). The patient can be a new patient or one of the first set.

At step S110, a gene expression profile is obtained for each of the five or more, for example, 5, 6, 7, 8, 9, 10, 11, 12, 13 or all, immune defense response genes, e.g., by performing PCR on the biological sample.

At step S112, a prediction of the radiotherapy response based on the gene expression profiles for the five or more immune defense response genes is determined for the patient using the regression function. This will be described in more detail later in the description.

At S114, a therapy recommendation may be provided, e.g., to the patient or his or her guardian, to a doctor, or to another healthcare worker, based on the prediction. To this end, the prediction may be categorized into one of a predefined set of risk groups, based on the value of the prediction. In one particular realization, the prediction of the radiotherapy response may be negative or positive for the effectiveness of the radiotherapy. If the prediction is negative, the recommended therapy may comprise one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

The method ends at S116.

In one embodiment, the gene expression profiles at steps S104 and S110 are determined by detecting mRNA expression using five or more primers and/or probes and/or five or more sets thereof.

In an alternative, the function for assigning the prediction of the radiotherapy response that is determined in step S106 is not based on a regression function but the gene expression profiles of the five or more immune defense response genes are combined with a sum function that sums discretized gene expression profiles of the five or more immune defense response genes, wherein the gene expression profiles are discretized using respective cut-offs that had been derived from the population of prostate cancer subjects. In one particular realization, the sum function is determined as specified in Eq. (2) above. In step S112, the prediction of the radiotherapy response is then determined for the patient using the sum function.

The immune system interacts in a strong manner with prostate cancer, both on a systemic level and in the tumour microenvironment. Immune defense response genes play a central role in the regulation of immune activity. Immune defense response genes may therefore provide information on the effectiveness of RT. However, which immune defense response genes may have predictive value in this application is extremely difficult to deduce from existing literature due to the many factors that influence the exact function of immune defense response genes.

We investigated the extent to which the expression of immune defense response genes in prostate cancer tissue correlates with the recurrence of disease after radical RT or SRT.

We have identified 14 immune defense response genes for which the degree of expression in prostate cancer tissue significantly correlates with mortality after SRT, in a cohort of 151 prostate cancer patients.

Based on the significant correlation with outcome after RT, we expect that the identified molecules will provide predictive value with regard to the effectiveness of radical RT and/or SRT.

RESULTS

## Cox Regression Analysis

We then set out to test whether the combination of these 14 immune defense response genes will exhibit more prognostic value. With Cox regression we modelled the expression levels of the 14 immune defense response genes to prostate cancer specific death after post-surgical salvage RT (IDR_model). We tested the model in Kaplan-Meier survival analysis.

The investigated patient group consisting of 538 subjects is a surgical cohort. All patients were undergoing prostatectomy to remove their primary prostate tumors. Approximately 30% of all patients experienced PSA relapse (or biochemical recurrence) within 10 years after prostate surgery. Therefore, many of those patients (#151) with PSA recurrence have undergone salvage radiation therapy (SRT) either alone or in combination with anti-androgen treatments as a secondary therapy to treat the recurring cancer cells. Despite this treatment, some 40 to 50% of these patients developed regional or distant metastases and some 25% died from prostate cancer within 5 to 10 years after the start of SRT.

The Cox regression function was derived as follows:

IDR_model:

$$(w_1 \cdot AIM2) + (w_2 \cdot APOBEC3A) + (w_3 \cdot CIAO1) + (w_4 \cdot DDX58) +$$
$$(w_5 \cdot DHX9) + (w_6 \cdot IFI16) + (w_7 \cdot IFIH1) + (w_8 \cdot IFIT1) + (w_9 \cdot$$
$$IFIT3) + (w_{10} \cdot LRRFIP1) + (w_{11} \cdot MYD88) + (w_{12} \cdot OAS1) + (w_{13} \cdot$$
$$TLR8) + (w_{14} \cdot ZBP1)$$

The details for the weights $w_1$ to $w_{14}$ are shown in the following TABLE 1.

TABLE 1: Variables and weights for the Cox regression model, i.e., the immune defense response model (IDR_model).

| Variable | Weights | |
|---|---|---|
| AIM2 | $w_1$ | -0,02116 |
| APOBEC3A | $w_2$ | -0,01195 |
| CIAO1 | $w_3$ | -0,02092 |
| DDX58 | $w_4$ | 0,009288 |
| DHX9 | $w_5$ | 0,002662 |
| IFI16 | $w_6$ | -0,01924 |
| IFIH1 | $w_7$ | 0,007922 |
| IFIT1 | $w_8$ | -0,06404 |
| IFIT3 | $w_9$ | 0,01869 |
| LRRFIP1 | $w_{10}$ | -0,005425 |
| MYD88 | $w_{11}$ | 0,002878 |

(continued)

| Variable | Weights | |
|---|---|---|
| OAS1 | $w_{12}$ | 0,001348 |
| TLR8 | $w_{13}$ | -0,04764 |
| ZBP1 | $w_{14}$ | -0,01494 |

**ROC Curve Analysis**

Next, we tested the logistic regression model as outlined above for their power to predict 5-year prostate cancer specific death (PCa Death) after start of salvage radiation therapy (SRT) due to post-surgical disease recurrence. The performance of the model was compared to the EAU BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019).

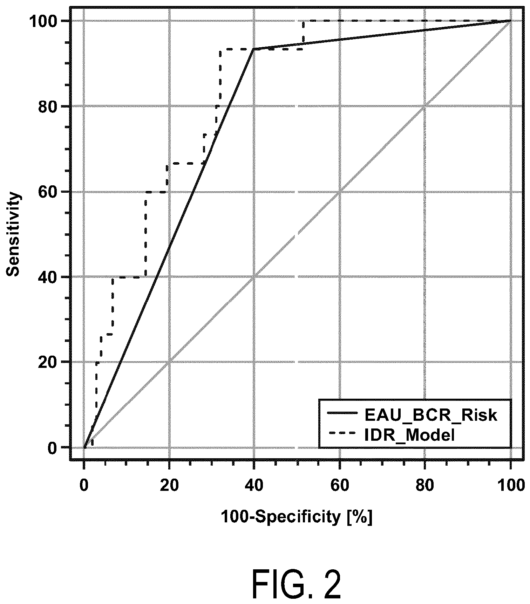

Fig. 2 shows a ROC curve analysis of two predictive models. The IDR_model (AUC=0,83) is the Cox regression model based on the 14 immune defense response genes. The EAU _BCR_Risk (AUC=0,76) is the EAU_BCR_Risk groups (European Association of Urology Biochemical Recurrence Risk groups).

**Kaplan-Meier Survival Analysis**

For Kaplan-Meier curve analysis, the Cox function of the risk model (TCR _signaling_model) was categorized into two sub-cohorts based on a cut-off (see description of figure below). The goal was to create patient classes by separating the classes with the median risk score as calculated from the IDR model for each patient with to some extent similar number of patients within the individual group.

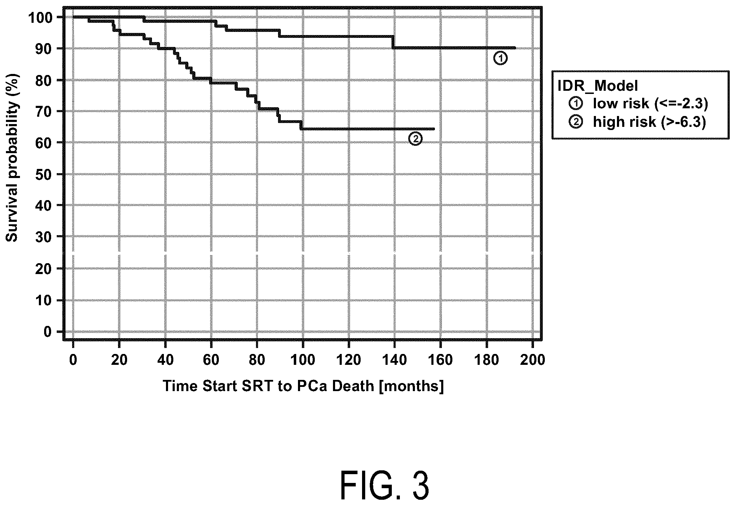

The patient classes represent an increasing risk to experience the tested clinical endpoints of time to prostate cancer specific death (Fig. 3) since the start of salvage radiation therapy (SRT) for the created risk model (IDR_model).

Fig. 3 shows a Kaplan-Meier curve of the IDR_model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR regression model using the value -2.3 as cut-off (logrank p=0.0001). The following supplementary list indicate the number of patients at risk for the TCR_signaling _model classes analyzed, i.e., the patients at risk at any time interval +20 months after surgery are shown: Low risk: 78, 77, 71, 68, 60, 46, 43, 22, 6, 3, 0; High risk: 73, 67, 59, 46, 35, 30, 28, 15, 0, 0, 0.

The Kaplan-Meier curve analysis as shown in Fig. 3 demonstrates the presence of different patient risk groups. The risk group of a patient is determined by the probability to suffer from the respective clinical endpoint (e.g., prostate cancer specific death) as calculated by the risk model IDR_model. Depending on the predicted risk of a patient (i.e., depending on in which risk group 1 or 2 the patient may belong) different types of interventions might be indicated.

**Immune Defense Response Score**

As an alternative to the regression model (IDR_model), we employed a combination of the gene expression profiles using a sum function (IDR_score). We extensively tested the model in Kaplan-Meier survival analysis.

The sum function was derived as follows:

IDR_score:

For each immune defense response gene i

$$if(\ EL(i) < \text{cut-off}(i)\ )$$

$$BEL(i) = 0$$

$$else$$

$$BEL(i) = 1$$

$$if\ (\ sum(\ BEL(i) < thresh\ )$$

$$IDR\_score = 0$$

$$else$$

$$IDR\_score = 1$$

The details for the cut-offs cut-off(i) and the threshold thresh are shown in the following TABLE 2.

TABLE 2: Variables, cut-offs and threshold for the sum model, i.e., the immune defense response score (IDR_score).

| Variable | Cut-off | Threshold |
|---|---|---|
| AIM2 | 33,44 | |
| APOBEC3A | 8,55 | |
| CIAO1 | 64,49 | |
| DDX58 | 45,87 | |
| DHX9 | 230,03 | |
| IFI16 | 53,56 | |
| IFIH1 | 99,63 | |
| IFIT1 | 14,57 | 1 |
| IFIT3 | 11,75 | |
| LRRFIP1 | 283,82 | |
| MYD88 | 23,03 | |
| OAS1 | 33,93 | |
| TLR8 | 9,89 | |
| ZBP1 | 20,16 | |

**Kaplan-Meier Survival Analysis**

For Kaplan-Meier curve analysis, the performance of the immune defense response score (IDR_score) was compared to the clinical risk score CAPRA-S (see Cooperberg M.R. et al., "The CAPRA-S score: A straightforward tool for improved prediction of outcomes after radical prostatectomy", Cancer, Vol. 117, No. 22, pages 5039-5046, 2011) and the EAU BCR risk groups (see Tilki D. et al., "External validation of the European Association of Urology Biochemical Recurrence Risk groups to predict metastasis and mortality after radical prostatectomy in a European cohort", Eur Urol, Vol. 75, No. 6, pages 896-900, 2019) for different patient groups and clinical endpoints.

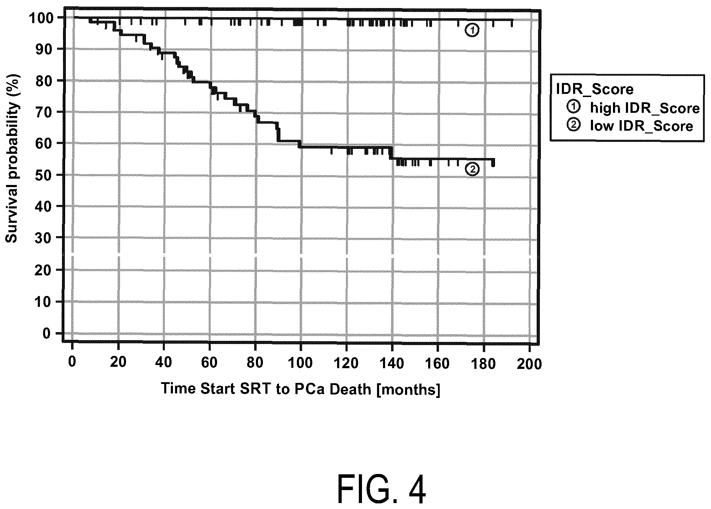

Fig. 4 shows a Kaplan-Meier curve analysis for men with post-surgical recurrent prostate cancer undergoing salvage radiation therapy (SRT). The total SRT patient sub-cohort of 151 men was stratified according to the level of the immune defense response score (IDR _score) into two cohorts (low score vs. high score). The clinical endpoint that was tested was prostate cancer specific death (PCa Death) after the start of SRT (logrank p<0.0001). The following supplementary lists indicate the number of patients at risk for the IDR_score classes analyzed, i.e., the patients at risk at any time interval +20 months after surgery are shown: High score: 76, 74, 70, 66, 59, 45, 41, 21, 3, 2, 0; Low score: 75, 70, 60, 48, 36, 31, 30, 16, 3, 1, 0.

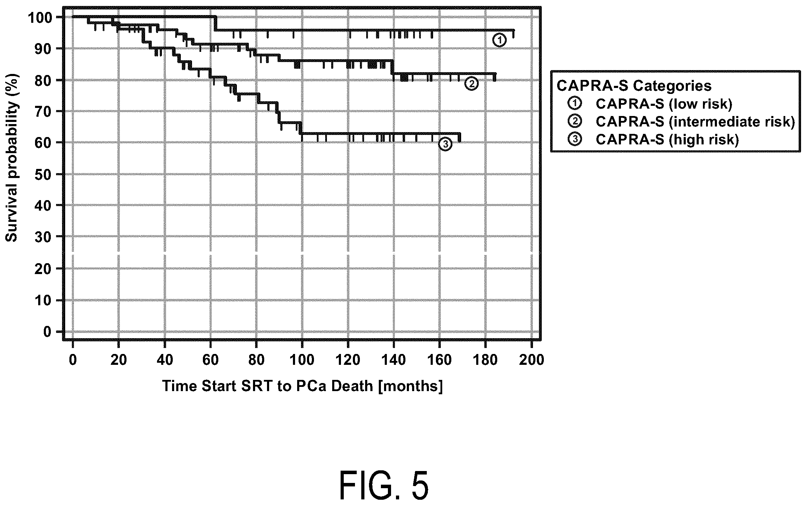

Fig. 5 shows a Kaplan-Meier curve analysis for men with post-surgical recurrent prostate cancer undergoing salvage radiation therapy (SRT). The total SRT patient sub-cohort of 151 men was stratified according to the level of the CARPA-S score categories (low, intermediate, high risk) into three cohorts. The clinical endpoint that was tested was prostate cancer specific death (PCa Death) after the start of SRT (logrank p=0.003). The following supplementary lists indicate the number of patients at risk for the CAPRA-S classes analyzed, i.e., the patients at risk at any time interval +20 months after surgery are shown: Low risk: 23, 23, 23, 23, 20, 18, 18, 12, 1, 1, 0; Intermediate risk: 76, 73, 66, 58, 50, 41, 38, 19, 4, 2, 0; High risk: 52, 48, 41, 33, 25, 17, 15, 6, 1, 0, 0.

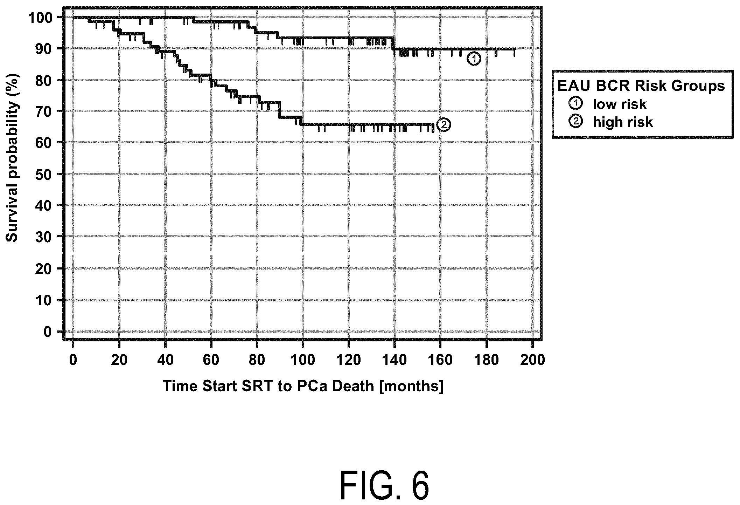

Fig. 6 shows a Kaplan-Meier curve analysis for men with post-surgical recurrent prostate cancer undergoing salvage radiation therapy (SRT). The total SRT patient sub-cohort of 151 men was stratified according to the level of the EAU BCR risk groups (EAU_BCR_Risk) into two cohorts (low risk vs. high risk). The clinical endpoint that was tested was prostate cancer specific death (PCa Death) after the start of SRT (logrank p=0.0001). The following supplementary lists indicate the number of patients at risk for the EAU_BCR_Risk classes analyzed, i.e., the patients at risk at any time interval +20 months after surgery are shown: Low risk: 72, 72, 69, 66, 58, 48, 45, 25, 6, 3, 0; High risk: 79, 72, 61, 48, 37, 28, 26, 12, 0, 0, 0.

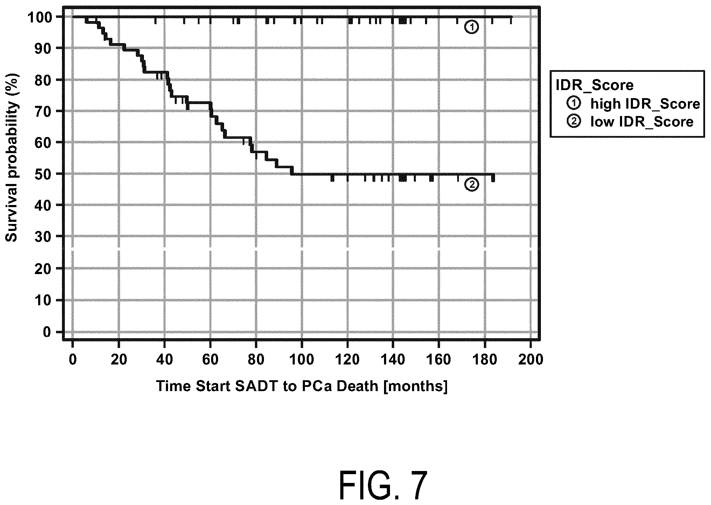

Fig. 7 shows a Kaplan-Meier curve analysis for men with post-surgical recurrent prostate cancer undergoing salvage androgen deprivation therapy (SADT). The total SADT patient sub-cohort of 106 men was stratified according to the level of the immune defense response score (IDR_score) into two cohorts (low score vs. high score). The clinical endpoint that was tested was prostate cancer specific death (PCa Death) after the start of SADT (logrank p<0.0001). The following supplementary lists indicate the number of patients at risk for the IDR_score classes analyzed, i.e., the patients at risk at any time interval +20 months after surgery are shown: High score: 48, 47, 46, 43, 38, 29, 27, 17, 3, 2, 0; Low score: 58, 52, 43, 33, 24, 21, 19, 13, 2, 1, 0.

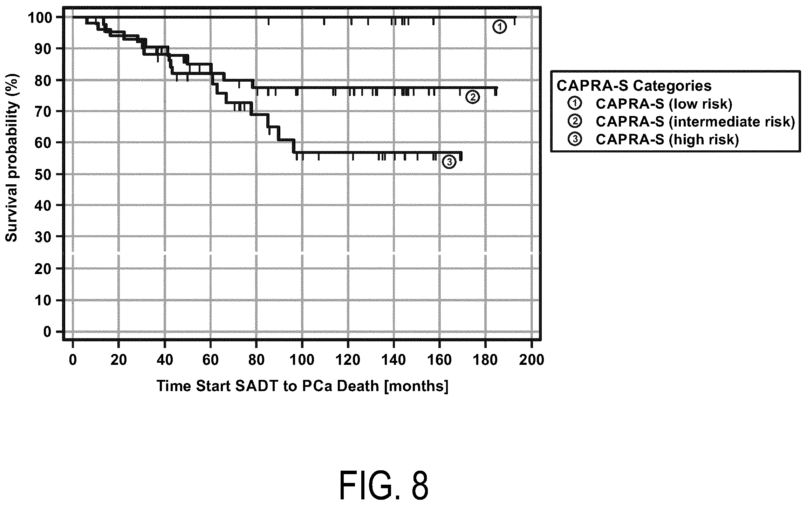

Fig. 8 shows a Kaplan-Meier curve analysis for men with post-surgical recurrent prostate cancer undergoing salvage androgen deprivation therapy (SADT). The total SADT patient sub-cohort of 106 men was stratified according to the level of the CARPA-S score categories (low, intermediate, high risk) into three cohorts. The clinical endpoint that was tested was prostate cancer specific death (PCa Death) after the start of SADT (logrank p=0.05). The following supplementary lists indicate the number of patients at risk for the CAPRA-S classes analyzed, i.e., the patients at risk at any time interval +20 months after surgery are shown: Low risk: 11, 11, 11, 11, 11, 10, 9, 6, 1, 1, 0; Intermediate risk: 51, 48, 44, 38, 33, 28, 26, 18, 3, 2, 0; High risk: 44, 40, 34, 27, 18, 12, 11, 6, 1, 0, 0.

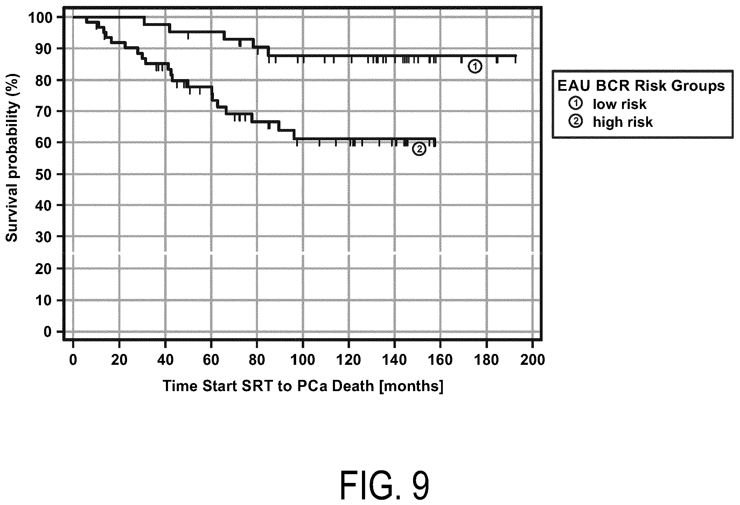

Fig. 9 shows a Kaplan-Meier curve analysis for men with post-surgical recurrent prostate cancer undergoing salvage androgen deprivation therapy (SADT). The total SADT patient sub-cohort of 106 men was stratified according to the level of the EAU BCR risk groups (EAU_BCR_Risk) into two cohorts (low risk vs. high risk). The clinical endpoint that was tested was prostate cancer specific death (PCa Death) after the start of SADT (logrank p=0.004). The following supplementary lists indicate the number of patients at risk for the EAU_BCR_Risk classes analyzed, i.e., the patients at risk at any time interval +20 months after surgery are shown: Low risk: 43, 43, 42, 40, 35, 29, 27, 19, 5, 3, 0; High risk: 63, 56, 47, 36, 27, 21, 19, 11, 0, 0, 0.

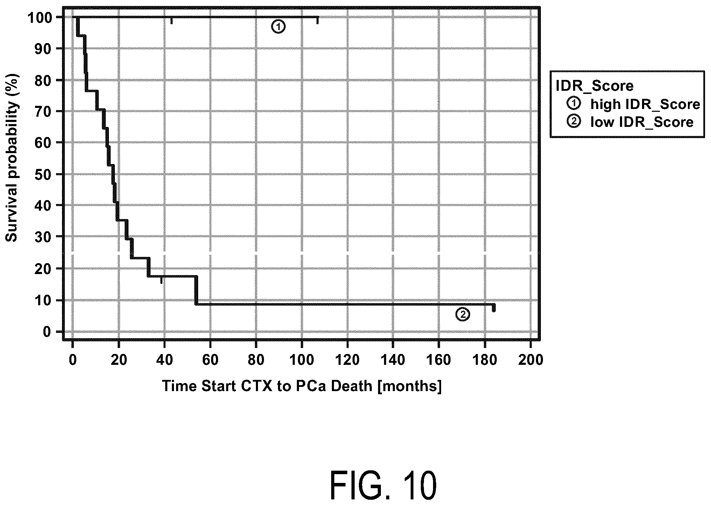

Fig. 10 shows a Kaplan-Meier curve analysis for men with post-surgical recurrent prostate cancer undergoing cytotoxic therapy (CTX). The total SADT patient sub-cohort of 10 men was stratified according to the level of the immune defense response score (IDR_score) into two cohorts (low score vs. high score). The clinical endpoint that was tested was prostate cancer specific death (PCa Death) after the start of CTX (logrank p=0.005). The following supplementary lists indicate the number of patients at risk for the IDR_score classes analyzed, i.e., the patients at risk at any time interval +20 months after surgery are shown: High score: 2, 2, 2, 1, 1, 1, 0, 0, 0, 0, 0; Low score: 17, 6, 2, 1, 1, 1, 1, 1, 1, 1, 0.

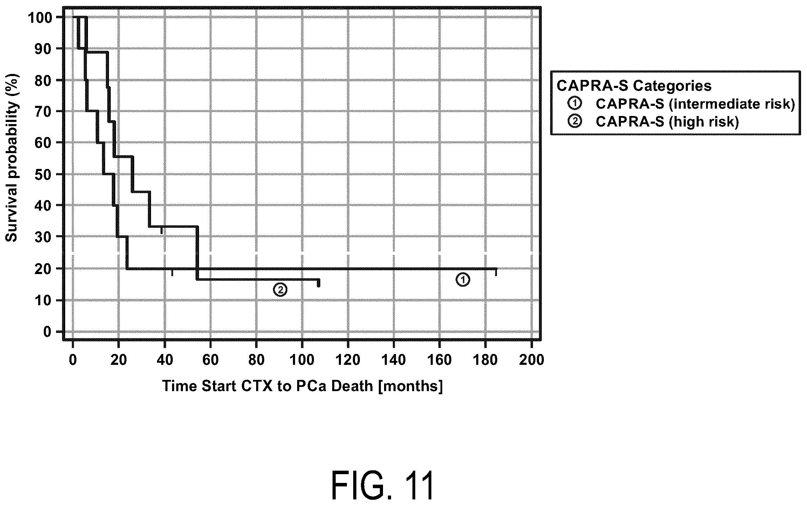

Fig. 11 shows a Kaplan-Meier curve analysis for men with post-surgical recurrent prostate cancer undergoing cytotoxic therapy (CTX). The total SADT patient sub-cohort of 19 men was stratified according to the level of the CARPA-S score categories (low, intermediate, high risk) into three cohorts. (In this example, there were no patients in the low risk class.) The clinical endpoint that was tested was prostate cancer specific death (PCa Death) after the start of CTX (logrank p=0.38). The following supplementary lists indicate the number of patients at risk for the CAPRA-S classes analyzed, i.e., the patients at risk at any time interval +20 months after surgery are shown: Low risk: -; Intermediate risk: 10, 3, 2, 1, 1, 1, 1, 1, 1, 1, 0; High risk: 9, 5, 2, 1, 1, 1, 0, 0, 0, 0, 0.

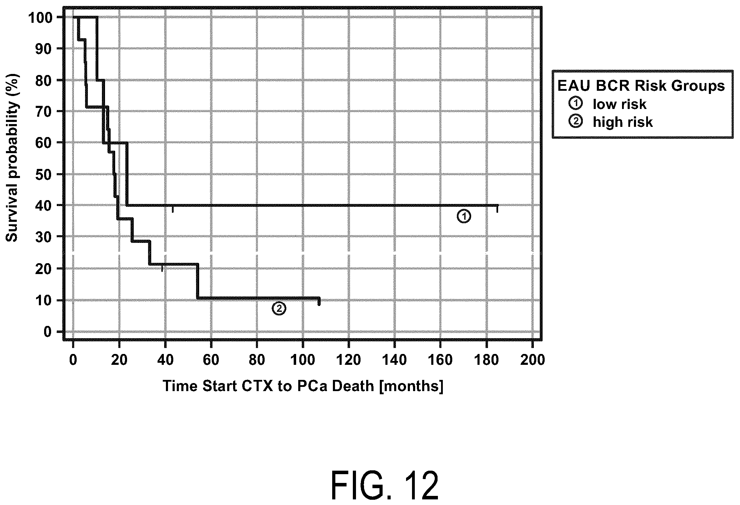

Fig. 12 shows a Kaplan-Meier curve analysis for men with post-surgical recurrent prostate cancer undergoing cytotoxic therapy (CTX). The total SADT patient sub-cohort of 19 men was stratified according to the level of the EAU BCR risk groups (EAU_BCR_Risk) into two cohorts (low risk vs. high risk). The clinical endpoint that was tested was prostate cancer specific death (PCa Death) after the start of CTX (logrank p=0.4). The following supplementary lists indicate the number of patients at risk for the EAU_BCR_Risk classes analyzed, i.e., the patients at risk at any time interval +20 months after surgery are shown: Low risk: 5, 3, 2, 1, 1, 1, 1, 1, 1, 1, 0; High risk: 14, 5, 2, 1, 1, 1, 0, 0, 0, 0, 0.

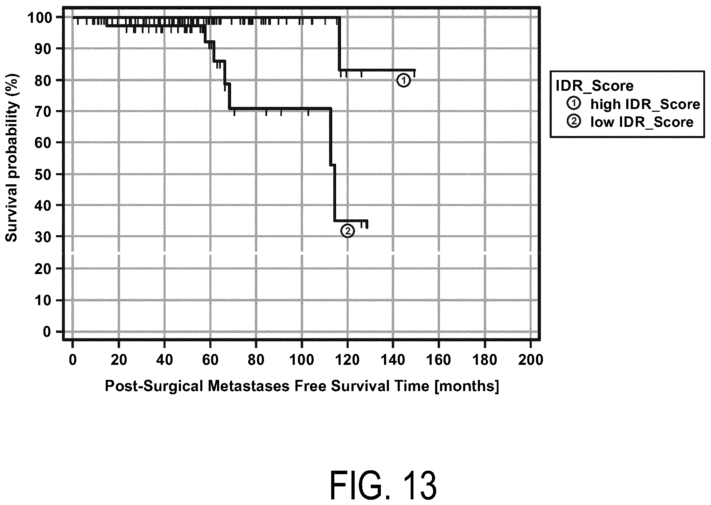

Fig. 13 shows a Kaplan-Meier curve analysis for men with post-surgical recurrent prostate cancer. The total patient cohort of 130 men was stratified according to the level of the immune defense response score (IDR_score) into two cohorts (low score vs. high score). The clinical endpoint that was tested was metastases-free survival after primary treatment (logrank p=0.0004). The following supplementary lists indicate the number of patients at risk for the IDR_score classes analyzed, i.e., the patients at risk at any time interval +20 months after surgery are shown: High score: 87, 81, 66, 36, 19, 11, 2, 1, 0; Low score: 43, 37, 29, 17, 8, 5, 2, 0, 0.

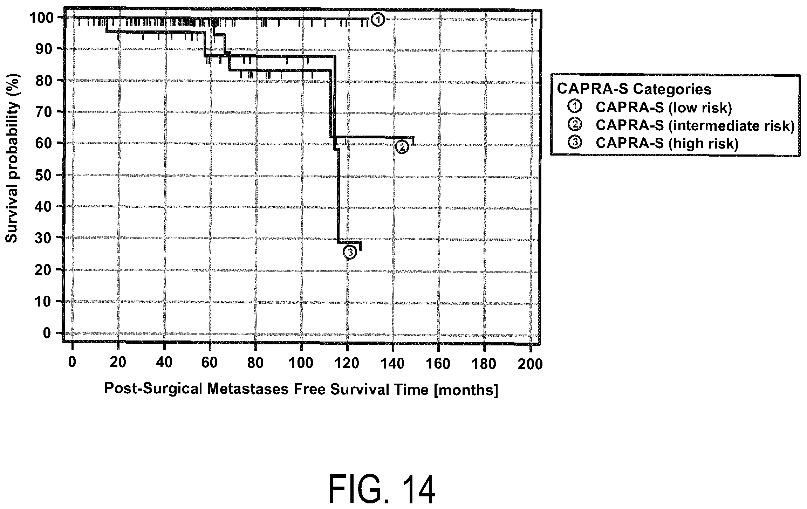

Fig. 14 shows a Kaplan-Meier curve analysis for men with post-surgical recurrent prostate cancer. The total patient cohort of 130 men was stratified according to the level of the CARPA-S score categories (low, intermediate, high risk) into three cohorts. The clinical endpoint that was tested was metastases-free survival after primary treatment (logrank p=0.04). The following supplementary lists indicate the number of patients at risk for the CAPRA-S classes analyzed, i.e., the patients at risk at any time interval +20 months after surgery are shown: Low risk: 64, 61, 46, 22, 12, 6, 2, 0, 0; Intermediate risk: 42, 37, 31, 21, 10, 6, 1, 1, 0; High risk: 23, 20, 18, 10, 5, 4, 1, 0, 0.

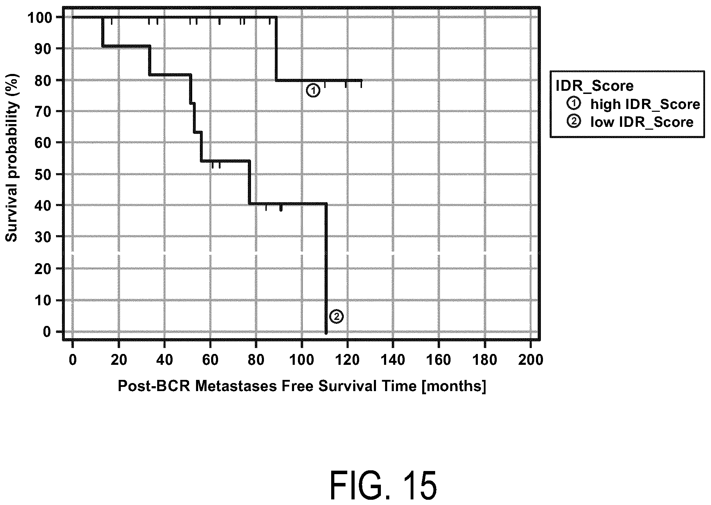

Fig. 15 shows a Kaplan-Meier curve analysis for men with post-surgical recurrent prostate cancer. The total patient cohort of 130 men was stratified according to the level of the immune defense response score (IDR_score) into two cohorts (low score vs. high score). The clinical endpoint that was tested was metastases-free survival after post-surgical biochemical recurrence (BCR) (logrank p=0.004). The following supplementary lists indicate the number of patients at risk for the IDR_score classes analyzed, i.e., the patients at risk at any time interval +20 months after surgery are shown: High score: 15, 14, 12, 10, 6, 4, 1, 0; Low score: 11, 10, 9, 6, 3, 1, 0, 0.

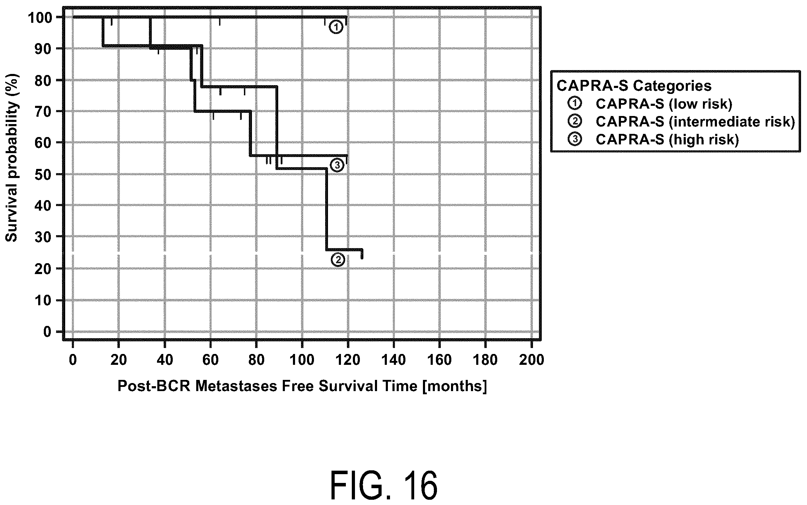

Fig. 16 shows a Kaplan-Meier curve analysis for men with post-surgical recurrent prostate cancer. The total patient cohort of 130 men was stratified according to the level of the CARPA-S score categories (low, intermediate, high risk) into three cohorts. The clinical endpoint that was tested was metastases-free survival after post-surgical biochemical recurrence (BCR) (logrank p=0.39). The following supplementary lists indicate the number of patients at risk for the CAPRA-S classes analyzed, i.e., the patients at risk at any time interval +20 months after surgery are shown: Low risk: 4, 4, 3, 3, 2, 2, 0, 0; Intermediate risk: 11, 10, 9, 7, 4, 1, 0, 0; High risk: 11, 10, 9, 6, 3, 2, 1, 0.

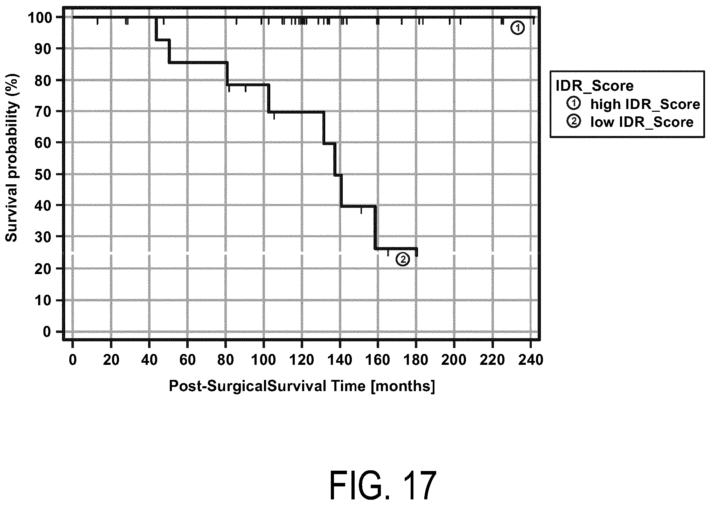

Fig. 17 shows a Kaplan-Meier curve analysis for men with post-surgical recurrent prostate cancer. The total patient cohort of 56 men was stratified according to the level of the immune defense response score (IDR_score) into two cohorts (low score vs. high score). The clinical endpoint that was tested was overall survival after primary treatment (logrank p<0.0001). The following supplementary lists indicate the number of patients at risk for the IDR_score classes analyzed, i.e., the patients at risk at any time interval +20 months after surgery are shown: High score: 42, 41, 39, 37, 37, 35, 26, 15, 9, 8, 5, 4, 1, 0; Low score: 14, 14, 14, 12, 11, 9, 7, 4, 2, 0, 0, 0, 0.

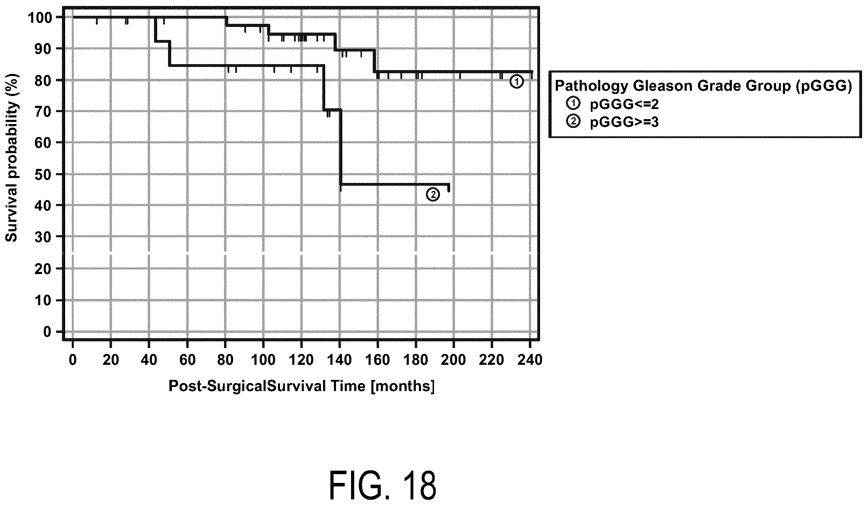

Fig. 18 shows a Kaplan-Meier curve analysis for men with post-surgical recurrent prostate cancer. The total patient cohort of 56 men was stratified according to the level of the pathology Gleason grade group into two cohorts (pGGG≤2 vs. pGGG≥3). The clinical endpoint that was tested was overall survival after primary treatment (logrank p<0.02). The following supplementary lists indicate the number of patients at risk for the pGGG classes analyzed, i.e., the patients at risk at any time interval +20 months after surgery are shown: pGGG≤2: 42, 42, 40, 38, 37, 35, 26, 18, 10, 7, 5, 4, 1, 0; pGGG≥3: 14, 13, 13, 11, 11, 9, 7, 1, 1, 1, 0, 0, 0.

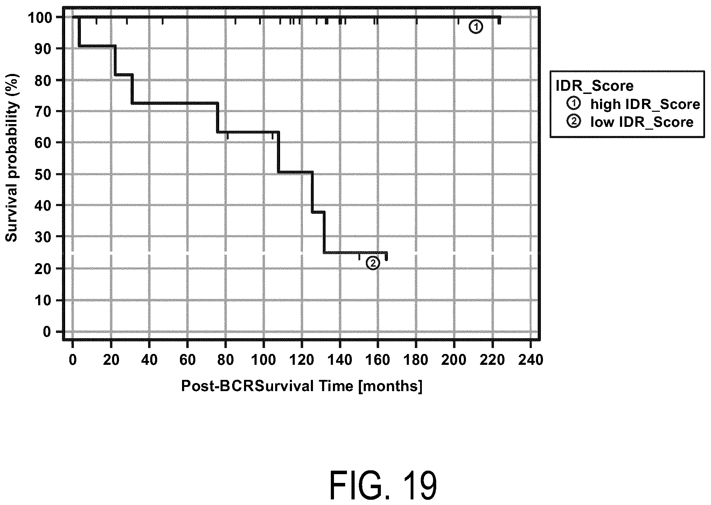

Fig. 19 shows a Kaplan-Meier curve analysis for men with post-surgical recurrent prostate cancer. The total patient cohort of 56 men was stratified according to the level of the immune defense response score (IDR_score) into two cohorts (low score vs. high score). The clinical endpoint that was tested was overall survival after post-surgical biochemical recurrence (BCR) (logrank p<0.0001). The following supplementary lists indicate the number of patients at risk for the IDR_score classes analyzed, i.e., the patients at risk at any time interval +20 months after surgery are shown: High score: 23, 22, 21, 20, 20, 18, 14, 10, 5, 5, 4, 3, 0; Low score: 11, 10, 8, 8, 7, 6, 4, 2, 1, 0, 0, 0.

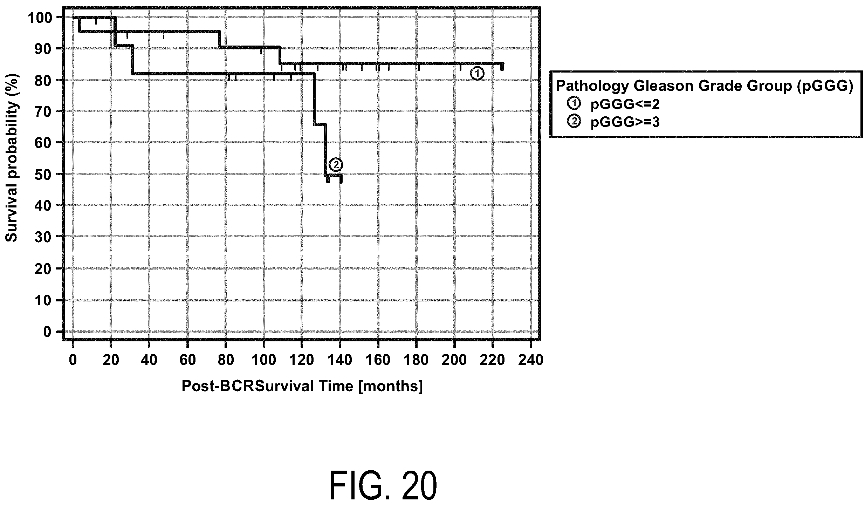

Fig. 20 shows a Kaplan-Meier curve analysis for men with post-surgical recurrent prostate cancer. The total patient cohort of 56 men was stratified according to the level of the pathology Gleason grade group into two cohorts (pGGG≤2 vs. pGGG≥3). The clinical endpoint that was tested was overall survival after post-surgical biochemical recurrence (BCR) (logrank p=0.13). The following supplementary lists indicate the number of patients at risk for the pGGG classes analyzed, i.e., the patients at risk at any time interval +20 months after surgery are shown: pGGG≤2: 22, 21, 20, 19, 18, 17, 13, 12, 6, 5, 4, 3, 0; pGGG≥3: 12, 11, 9, 9, 9, 7, 5, 0, 0, 0, 0, 0, 0.

The data presented in Figs. 2 to 20 demonstrate that the measurement and combination of immune defense response genes provide a means to define patient groups with different risks to die from prostate cancer after post-surgical disease recurrence and start of secondary therapies like radiation therapy. Furthermore, the presented data shows that a selection of 14 of these immune defense genes can separate patient groups with different risks of progressive disease in multiple independent data sets.

**Further results**

This section shows additional results for Cox regression models based on only five and six of the identified immune defense response genes, respectively. In total, eight different 5 gene models and four different 6 gene models were tested. The details for the weights are shown in the following TABLES 3 and 4, respectively.

TABLE 3: Variables and weights for the 5 gene Cox regression models, i.e., the eight immune defense response 5 gene models (IDR _5.1_model to IDR_5.8_model); NA - not available.

| Variable | IDR regression model | | | | | | | |
|---|---|---|---|---|---|---|---|---|
| | 5.1 | 5.2 | 5.3 | 5.4 | 5.5 | 5.6 | 5.7 | 5.8 |
| AIM2 | 0.1316 | NA | NA | NA | NA | NA | 0.1032 | NA |
| APOBEC3A | NA | -0.1672 | NA | NA | -0.4507 | -0.4286 | NA | NA |
| CIAO1 | -0.3169 | NA | NA | -0.2667 | NA | -0.3709 | NA | -0.2374 |
| DDX58 | NA | NA | 0.01555 | NA | 0.00885 | NA | NA | NA |
| DHX9 | NA | NA | NA | NA | NA | NA | -0.2428 | -0.1563 |
| IFI16 | NA | 0,2713 | NA | NA | 0.2449 | 0.2908 | NA | 0.1626 |
| IFIH1 | 0.3405 | NA | 0.4204 | 0.3212 | 0.2917 | 0.3821 | 0.1787 | NA |
| IFIT1 | NA | NA | -0.2197 | NA | NA | 0.0953 | NA | -0.2132 |
| IFIT3 | NA | -0.0097 | -0.0132 | 0.03645 | -0.0422 | NA | NA | NA |
| LRRFIP1 | -0.1206 | NA | NA | NA | NA | NA | NA | 0.03902 |
| MYD88 | NA | -0.1838 | NA | -0.1297 | NA | NA | NA | NA |
| OAS1 | 0.1109 | NA | NA | NA | NA | NA | 0.07908 | NA |
| TLR8 | NA | -0.3846 | -0.6323 | NA | NA | NA | NA | NA |
| ZBP1 | NA | NA | NA | -0.006 | NA | NA | 0.1237 | NA |

TABLE 4: Variables and weights for the 6 gene Cox regression models, i.e., the four immune defense response 6 gene models (IDR_6.1_model to IDR_6.4_model); NA - not available.

| Variable | IDR regression model | | | |
|---|---|---|---|---|
| | 6.1 | 6.2 | 6.3 | 6.4 |
| AIM2 | 0.09664 | NA | NA | NA |
| APOBEC3A | NA | -0.1792 | NA | -0.2547 |
| CIAO1 | -0.3408 | NA | NA | NA |
| DDX58 | NA | 0.03933 | 0.0161 | 0.00979 |
| DHX9 | NA | NA | NA | NA |
| IFI16 | 0.1836 | 0.2672 | NA | 0.2167 |
| IFIH1 | 0.3405 | NA | 0.3478 | 0.4286 |
| IFIT1 | NA | NA | -0.3142 | -0.1954 |
| IFIT3 | NA | -0.0158 | -0.0349 | NA |
| LRRFIP1 | -01497 | NA | NA | NA |
| MYD88 | NA | -0.1865 | NA | NA |
| OAS1 | 0.04808 | NA | NA | NA |
| TLR8 | NA | -0.3911 | -0.6472 | -0.576 |
| ZBP1 | NA | NA | 0.2645 | NA |

Fig. 21 shows a Kaplan-Meier curve of the IDR_5.1 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.1 model using the value -0.1 as cut-off (logrank p=0.003; HR=3.3; CI=1.5-7.2). The following supplementary list indicate the number of patients at risk for the IDR_5.1 model classes analyzed, i.e., the patients at risk at any time interval +20 months after surgery are shown: Low risk: 53, 49, 43, 34, 22, 13, 4, 3, 0; High risk: 132, 114, 88, 64, 38, 20, 11, 5, 0.

Fig. 22 shows a Kaplan-Meier curve of the IDR_5.2 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.2 model using the value 0.16 as cut-off (logrank p=0.004; HR=3.2; CI=1.5-7.0). The following supplementary list indicate the number of patients at risk for the IDR_5.2 model classes analyzed, i.e., the patients at risk at any time interval +20 months after surgery are shown: Low risk: 90, 81, 67, 51, 34, 19, 7, 3, 0; High risk: 95, 82, 64, 47, 26, 14, 8, 5, 0.

Fig. 23 shows a Kaplan-Meier curve of the IDR_5.3 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.3 model using the value 0.2 as cut-off (logrank p=0.005; HR=3.1; CI=1.4-6.9). The following supplementary list indicate the number of patients at risk for the IDR_5.3 model classes analyzed, i.e., the patients at risk at any time interval +20 months after surgery are shown: Low risk: 96, 86, 71, 56, 34, 21, 8, 5, 0; High risk: 89, 77, 60, 42, 26, 12, 7, 3, 0.

Fig. 24 shows a Kaplan-Meier curve of the IDR_5.4 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.4 model using the value -0.1 as cut-off (logrank p=0.007; HR=2.0; CI=1.3-6.4). The following supplementary list indicate the number of patients at risk for the IDR_5.4 model classes analyzed, i.e., the patients at risk at any time interval +20 months after surgery are shown: Low risk: 90, 80, 67, 53, 32, 17, 8, 3, 0; High risk: 95, 83, 64, 45, 28, 16, 7, 5, 0.

Fig. 25 shows a Kaplan-Meier curve of the IDR_5.5 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.5 model using the value -0.1 as cut-off (logrank p=0.004; HR=3.2; CI=1.5-7.0). The following supplementary list indicate the number of patients at risk for the IDR_5.5 model classes analyzed, i.e., the patients at risk at any time interval +20 months after surgery are shown: Low risk: 67, 63, 56, 43, 26, 13, 6, 2, 0; High risk: 118, 100, 75, 55, 34, 20, 9, 6, 0.

Fig. 26 shows a Kaplan-Meier curve of the IDR_5.6 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.6 model using the value -0.12 as cut-off (logrank p=0.0004; HR=4.1; CI=1.9-9.0). The following supplementary list indicate the number of patients at risk for the IDR_5.6 model classes analyzed, i.e., the patients at risk at any time interval +20 months after surgery are shown: Low risk: 66, 62, 55, 42, 25, 12, 6, 2, 0; High risk: 119, 101, 76, 56, 35, 21, 9, 6, 0.

Fig. 27 shows a Kaplan-Meier curve of the IDR_5.7 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.7 model using the value 0.1 as cut-off (logrank p=0.01; HR=2.9; CI=1.3-6.7). The following supplementary list indicate the number of patients at risk for the IDR_5.7 model classes analyzed, i.e., the patients at risk at any time interval +20 months after surgery are shown: Low risk: 117, 194, 84, 67, 44, 21, 9, 5, 0; High risk: 68, 59, 47, 31, 16, 12, 6, 3, 0.

Fig. 28 shows a Kaplan-Meier curve of the IDR_5.8 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_5.8 model using the value 0.0 as cut-off (logrank p=0.005; HR=3.0; CI=1.4-6.7). The following supplementary list indicate the number of patients at risk for the IDR_5.8 model classes analyzed, i.e., the patients at risk at any time interval +20 months after surgery are shown: Low risk: 88, 79, 68, 54, 33, 17, 7, 3, 0; High risk: 97, 84, 63, 44, 27, 16, 8, 5, 0.

Fig. 29 shows a Kaplan-Meier curve of the IDR_6.1 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_6.1 model using the value -0.1 as cut-off (logrank p=0.02; HR=2.5; CI=1.2-5.6). The following supplementary list indicate the number of patients at risk for the IDR_6.1 model classes analyzed, i.e., the patients at risk at any time interval +20 months after surgery are shown: Low risk: 67, 62, 53, 42, 23, 15, 7, 5, 0; High risk: 118, 101, 78, 56, 37, 18, 8, 3, 0.

Fig. 30 shows a Kaplan-Meier curve of the IDR_6.2 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_6.2 model using the value 0.17 as cut-off (logrank p=0.004; HR=3.2; CI=1.5-7.1). The following supplementary list indicate the number of patients at risk for the IDR_6.2 model classes analyzed, i.e., the patients at risk at any time interval +20 months after surgery are shown: Low risk: 90, 81, 67, 51, 34, 19, 7, 3, 0; High risk: 95, 82, 64, 47, 26, 14, 8, 5, 0.

Fig. 31 shows a Kaplan-Meier curve of the IDR_6.3 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_6.3 model using the value 0.16 as cut-off (logrank p=0.008; HR=2.9; CI=1.3-6.4). The following supplementary list indicate the number of patients at risk for the IDR_6.3 model classes analyzed, i.e., the patients at risk at any time interval +20 months after surgery are shown: Low risk: 92, 85, 71, 56, 32, 19, 7, 4, 0; High risk: 93, 78, 60, 42, 28, 14, 8, 4, 0.

Fig. 32 shows a Kaplan-Meier curve of the IDR_6.4 model. The clinical endpoint that was tested was the time to prostate cancer specific death (PCa Death) after the start of salvage radiation therapy (SRT) due to post-surgical disease recurrence. Patients were stratified into two cohorts (low vs. high) according to their risk to experience the clinical endpoint as predicted by the IDR_6.4 model using the value 0.1 as cut-off (logrank p=0.006; HR=3.0; CI=1.4-6.6). The following supplementary list indicate the number of patients at risk for the IDR_6.4 model classes analyzed, i.e., the patients at risk at any time interval +20 months after surgery are shown: Low risk: 88, 79, 66, 53, 31, 18, 7, 5, 0; High risk: 97, 84, 65, 45, 29, 15, 8, 3, 0.

The Kaplan-Meier analysis as shown in Figs. 21 to 32 demonstrates that different patient risk groups can also be distinguished using risk models that are only based on a subset of the identified immune defense response genes, for example, five or six of the genes.

## Discussion

The effectiveness of both radical RT and SRT for localized prostate cancer is limited, resulting in disease progression and ultimately death of patients, especially for those at high risk of recurrence. The prediction of the therapy outcome is very complicated as many factors play a role in therapy effectiveness and disease recurrence. It is likely that important factors have not yet been identified, while the effect of others cannot be determined precisely. Multiple clinico-pathological measures are currently investigated and applied in a clinical setting to improve response prediction and therapy selection, providing some degree of improvement. Nevertheless, a strong need remains for better prediction of the response to radical RT and to SRT, in order to increase the success rate of these therapies.

We have identified molecules of which expression shows a significant relation to mortality after radical RT and SRT and therefore are expected to improve the prediction of the effectiveness of these treatments. An improved prediction of effectiveness of RT for each patient be it in the radical or the salvage setting, will improve therapy selection and potentially survival. This can be achieved by 1) optimizing RT for those patients where RT is predicted to be effective (e.g. by dose escalation or a different starting time) and 2) guiding patients where RT is predicted not to be effective to an alternative, potentially more effective form of treatment. Further, this would reduce suffering for those patients who would be spared ineffective therapy and would reduce cost spent on ineffective therapies.

Other variations to the disclosed realizations can be understood and effected by those skilled in the art in practicing the claimed invention, from a study of the drawings, the disclosure, and the appended claims.

In aspects of the invention that are not currently claimed, a gene expression profile for each of five or more, for example, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 or all, immune defense response genes selected from the group consisting of: APOBEC3A, AIM2, APOBEC3B, CASP1, CIAO1, CTNNB1, CXCL10, CXCL9, DDX41, DDX58, DDX60, DHX36, DHX58, DHX9, GBP1, HERC5, HIST2H2BE, IFI16, IFI44L, IFIH1, IFIT1, IFIT1B, IFIT2, IFIT3, IFITM1, IFITM3, IL1B, IRF3, IRF5, IRF7, ISG15, ITGAX, LILRB1, LRRFIP1, MAVS, MB21D1, MX1, MX2, MYD88, NLRP3, NOD2, OAS1, OAS3, OASL, OPRK1, POLR3A, PTPRC, PYCARD, RSAD2, STAT1, TBK1, TICAM1, TLR2, TLR3, TLR4, TLR7, TLR8, TLR9, TMEM173, TRIM22, and ZBP1, may be used for determining the prediction of the radiotherapy response.

In the claims, the word "comprising" does not exclude other elements or steps, and the indefinite article "a" or "an" does not exclude a plurality.

One or more steps of the method illustrated in Fig. 1 may be implemented in a computer program product that may be executed on a computer. The computer program product may comprise a non-transitory computer-readable recording medium on which a control program is recorded (stored), such as a disk, hard drive, or the like. Common forms of non-transitory computer-readable media include, for example, floppy disks, flexible disks, hard disks, magnetic tape, or any other magnetic storage medium, CD-ROM, DVD, or any other optical medium, a RAM, a PROM, an EPROM, a FLASH-EPROM, or other memory chip or cartridge, or any other non-transitory medium from which a computer can read and use.

Alternatively, the one or more steps of the method may be implemented in transitory media, such as a transmittable carrier wave in which the control program is embodied as a data signal using transmission media, such as acoustic or light waves, such as those generated during radio wave and infrared data communications, and the like.

The exemplary method may be implemented on one or more general purpose computers, special purpose computer(s), a programmed microprocessor or microcontroller and peripheral integrated circuit elements, an ASIC or other integrated circuit, a digital signal processor, a hardwired electronic or logic circuit such as a discrete element circuit, a programmable logic device such as a PLD, PLA, FPGA, Graphical card CPU (GPU), or PAL, or the like. In general, any device, capable of implementing a finite state machine that is in turn capable of implementing the flowchart shown in Fig. 1, can be used to implement one or more steps of the method of risk stratification for therapy selection in a patient with prostate cancer is illustrated. As will be appreciated, while the steps of the method may all be computer implemented, in some embodiments one or more of the steps may be at least partially performed manually.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Any reference signs in the claims should not be construed as limiting the scope.

The invention relates to a method of predicting a response of a prostate cancer subject to radiotherapy, comprising determining or receiving the result of a determination of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject, determining, preferably by a processor, the prediction of the radiotherapy response based on the gene expression profiles for the five or more immune defense response genes, and optionally, providing the prediction or a therapy recommendation based on the prediction to a medical caregiver or the subject. Since the status of the immune system and of the immune microenvironment have an impact on therapy effectiveness, the ability to identify markers predictive for this effect might help to be better able to predict overall RT response. Immune defense response genes play a central role in the regulation of immune activity. The identified immune defense response genes were found to exhibit a significant correlation with outcome after RT, wherefore we expect that they will provide predictive value with regard to the effectiveness of radical RT and/or SRT.

**Claims**

1. A method of predicting a response of a prostate cancer subject to radiotherapy, comprising:

   - determining a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
   - determining, the prediction of the radiotherapy response based on the gene expression profiles for the five or more immune defense response genes, and
   - optionally, providing the prediction or a therapy recommendation based on the prediction to a medical caregiver or the subject.

2. A method of predicting a response of a prostate cancer subject to radiotherapy, comprising:

   - receiving the result of a determination of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
   - determining, by a processor, the prediction of the radiotherapy response based on the gene expression profiles for the five or more immune defense response genes, and
   - optionally, providing the prediction or a therapy recommendation based on the prediction to a medical caregiver or the subject.

3. A method of predicting a response of a prostate cancer subject to radiotherapy, comprising:

   - determining or receiving the result of a determination of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
- determining, by a processor, the prediction of the radiotherapy response based on the gene expression profiles for the five or more immune defense response genes, and
- optionally, providing the prediction or a therapy recommendation based on the prediction to a medical caregiver or the subject.

4. The method as defined in claim 1, 2 or 3, wherein the five or more immune defense response genes comprise six or more of the immune defense response genes.

5. The method as defined in any of claims 1 -3, wherein the five or more immune defense response genes comprise nine or more of the immune defense response genes.

6. The method as defined in any of claims 1 to 5, wherein the five or more immune defense response genes comprise twelve or more, preferably, all of the immune defense response genes.

7. The method as defined in any of claims 1 to 6, wherein the determining of the prediction of the radiotherapy response comprises combining the gene expression profiles for five or more, for example, 5, 6, 7, 8, 9, 10, 11, 12, 13 or all, of the immune defense response genes with a regression function that had been derived from a population of prostate cancer subjects.

8. The method as defined in any of claim 1-3, wherein the determining of the prediction of the radiotherapy response comprises combining the gene expression profiles for five or more, for example, 5, 6, 7, 8, 9, 10, 11, 12, 13 or all, of the immune defense response genes with a sum function that sums discretized gene expression profiles of the five or more immune defense response genes, wherein the gene expression profiles are discretized using respective cut-offs that had been derived from a population of prostate cancer subjects.

9. The method as defined in any of claims 1 to 8, wherein the biological sample is obtained from the subject before the start of the radiotherapy.

10. The method as defined in any of claims 1 to 9, wherein the radiotherapy is radical radiotherapy or salvage radiotherapy.

11. The method as defined in any of claims 1 to 10, wherein the prediction of the radiotherapy response is negative or positive for the effectiveness of the radiotherapy, wherein a therapy is recommended based on the prediction and, if the prediction is negative, the recommended therapy comprises one or more of: (i) radiotherapy provided earlier than is the standard; (ii) radiotherapy with an increased radiation dose; (iii) an adjuvant therapy, such as androgen deprivation therapy; and iv) an alternative therapy that is not a radiation therapy.

12. An apparatus for predicting a response of a prostate cancer subject to radiotherapy, comprising:

- an input adapted to receive data indicative of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,
- a processor adapted to determine the prediction of the radiotherapy response based on the gene expression profiles for the five or more immune defense response genes, and
- optionally, a providing unit adapted to provide the prediction or a therapy recommendation based on the prediction to a medical caregiver or the subject.

13. A computer program product comprising instructions which, when the program is executed by a computer, cause the computer to carry out a method comprising:

- receiving data indicative of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, said gene expression profiles being determined in a biological sample obtained from the subject,

- determining the prediction of the radiotherapy response based on the gene expression profiles for the five or more immune defense response genes, and
- optionally, providing the prediction or a therapy recommendation based on the prediction to a medical caregiver or the subject.

14. A diagnostic kit for predicting a response of a prostate cancer subject to radiotherapy, comprising:

- at least five primers and/or probes for determining the gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in a biological sample obtained from the subject, and
- an apparatus as defined in claim 12 or a computer program product as defined in claim 13.

15. The use of the kit as defined in claim 14 in a method of predicting a response of a prostate cancer subject to radiotherapy.

16. A method for predicting a response of a prostate cancer subject to radiotherapy, comprising:

- receiving a biological sample obtained from a prostate cancer subject,
- using the kit as defined in claim 14 to determine a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP1, in the biological sample obtained from the subject.

17. Use of a gene expression profile for each of five or more, for example, 5, 6, 7, 8, 9, 10, 11, 12, 13 or all, immune defense response genes selected from the group consisting of: AIM2, APOBEC3A, CIAO1, DDX58, DHX9, IFI16, IFIH1, IFIT1, IFIT3, LRRFIP1, MYD88, OAS1, TLR8, and ZBP 1, in a method of predicting a response of a prostate cancer subject to radiotherapy, comprising:

- determining, by a processor, the prediction of the radiotherapy response based on the gene expression profiles for the five or more immune defense response genes, and
- optionally, providing the prediction or a therapy recommendation based on the prediction to a medical caregiver or the subject.

**Patentansprüche**

1. Verfahren zur Vorhersage einer Reaktion eines Prostatakrebspatienten auf eine Strahlentherapie, umfassend:

- Bestimmen eines Genexpressionsprofils für jedes von fünf oder mehr, beispielsweise 5, 6, 7, 8, 9, 10, 11, 12, 13 oder allen, Genen der Immunabwehrreaktion, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1, wobei die Genexpressionsprofile in einer vom Patienten erhaltenen biologischen Probe bestimmt werden,
- Bestimmen der Vorhersage der Strahlentherapiereaktion basierend auf den Genexpressionsprofilen für die fünf oder mehr Gene der Immunabwehrreaktion und
- optional Bereitstellen der Vorhersage oder einer auf der Vorhersage basierenden Therapieempfehlung an eine medizinische Fachkraft oder den Patienten.

2. Verfahren zur Vorhersage einer Reaktion eines Prostatakrebspatienten auf eine Strahlentherapie, umfassend:

- Empfangen des Ergebnisses einer Bestimmung eines Genexpressionsprofils für jedes von fünf oder mehr, beispielsweise 5, 6, 7, 8, 9, 10, 11, 12, 13 oder allen, Genen der Immunabwehrreaktion, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1, wobei die Genexpressionsprofile in einer vom Patienten erhaltenen biologischen Probe bestimmt wurden,
- Bestimmen der Vorhersage der Strahlentherapiereaktion durch einen Prozessor basierend auf den Genexpressionsprofilen für die fünf oder mehr Gene der Immunabwehrreaktion und
- optional Bereitstellen der Vorhersage oder einer auf der Vorhersage basierenden Therapieempfehlung an eine medizinische Fachkraft oder den Patienten.

3. Verfahren zur Vorhersage einer Reaktion eines Prostatakrebspatienten auf eine Strahlentherapie, umfassend:

- Bestimmen oder Empfangen des Ergebnisses einer Bestimmung eines Genexpressionsprofils für jedes von fünf oder mehr, beispielsweise 5, 6, 7, 8, 9, 10, 11, 12, 13 oder allen, Genen der Immunabwehrreaktion, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1, wobei die Genexpressionsprofile in einer vom Patienten erhaltenen biologischen Probe bestimmt wurden,
- Bestimmen der Vorhersage der Strahlentherapiereaktion durch einen Prozessor basierend auf den Genexpressionsprofilen für die fünf oder mehr Gene der Immunabwehrreaktion und
- optional Bereitstellen der Vorhersage oder einer auf der Vorhersage basierenden Therapieempfehlung an eine medizinische Fachkraft oder den Patienten.

4. Verfahren nach Anspruch 1, 2 oder 3, wobei die fünf oder mehr Gene der Immunabwehrreaktion sechs oder mehr Gene der Immunabwehrreaktion umfassen.

5. Verfahren nach einem der Ansprüche 1-3, wobei die fünf oder mehr Gene der Immunabwehrreaktion neun oder mehr Gene der Immunabwehrreaktion umfassen.

6. Verfahren nach einem der Ansprüche 1 bis 5, wobei die fünf oder mehr Gene der Immunabwehrreaktion zwölf oder mehr, bevorzugt alle Gene der Immunabwehrreaktion umfassen.

7. Verfahren nach einem der Ansprüche 1 bis 6, wobei das Bestimmen der Vorhersage der Strahlentherapiereaktion das Kombinieren der Genexpressionsprofile für fünf oder mehr, beispielsweise 5, 6, 7, 8, 9, 10, 11, 12, 13 oder alle der Gene der Immunabwehrreaktion mit einer Regressionsfunktion umfasst, die aus einer Population von Prostatakrebspatienten abgeleitet wurde.

8. Verfahren nach einem der Ansprüche 1-3, wobei das Bestimmen der Vorhersage der Strahlentherapiereaktion das Kombinieren der Genexpressionsprofile für fünf oder mehr, beispielsweise 5, 6, 7, 8, 9, 10, 11, 12, 13 oder alle der Gene der Immunabwehrreaktion mit einer Summenfunktion umfasst, die diskretisierte Genexpressionsprofile der fünf oder mehr Gene der Immunabwehrreaktion summiert, wobei die Genexpressionsprofile unter Verwendung jeweiliger Cut-offs diskretisiert werden, die aus einer Population von Patienten mit Prostatakrebs abgeleitet wurden.

9. Verfahren nach einem der Ansprüche 1 bis 8, wobei die biologische Probe vor Beginn der Strahlentherapie vom Patienten erhalten wird.

10. Verfahren nach einem der Ansprüche 1 bis 9, wobei es sich bei der Strahlentherapie um eine radikale Strahlentherapie oder eine Salvage-Strahlentherapie handelt.

11. Verfahren nach einem der Ansprüche 1 bis 10, wobei die Vorhersage der Strahlentherapiereaktion hinsichtlich der Wirksamkeit der Strahlentherapie negativ oder positiv ist, wobei eine Therapie basierend auf der Vorhersage empfohlen wird und, wenn die Vorhersage negativ ist, die empfohlene Therapie eine oder mehrere der folgenden Maßnahmen umfasst: (i) eine Strahlentherapie, die früher als üblich bereitgestellt wird; (ii) eine Strahlentherapie mit erhöhter Strahlendosis; (iii) eine adjuvante Therapie, wie etwa eine Androgendeprivationstherapie; und/oder iv) eine alternative Therapie, die keine Strahlentherapie ist.

12. Einrichtung zur Vorhersage einer Reaktion eines Prostatakrebspatienten auf eine Strahlentherapie, umfassend:

- einen Eingang, der dazu angepasst ist, Daten zu empfangen, die ein Genexpressionsprofil für jedes von fünf oder mehr, beispielsweise 5, 6, 7, 8, 9, 10, 11, 12, 13 oder alle, Gene der Immunabwehrreaktion anzeigen, die aus der Gruppe ausgewählt sind, die aus AIM2, APOBEC3A, CIAO1, DDX58, DHX9, IFI16, IFIH1, IFIT1, IFIT3, LRRFIP1, MYD88, OAS1, TLR8 und ZBP1 besteht, wobei die Genexpressionsprofile in einer vom Patienten erhaltenen biologischen Probe bestimmt werden,
- einen Prozessor, der dazu angepasst ist, die Vorhersage der Strahlentherapiereaktion basierend auf den Genexpressionsprofilen für die fünf oder mehr Gene der Immunabwehrreaktion zu bestimmen, und
- optional eine Bereitstellungseinheit, die dazu angepasst ist, die Vorhersage oder eine auf der Vorhersage basierende Therapieempfehlung an eine medizinische Fachkraft oder den Patienten bereitzustellen.

13. Computerprogrammprodukt, umfassend Anweisungen, die, wenn das Programm von einem Computer ausgeführt wird, den Computer veranlassen, ein Verfahren auszuführen, das Folgendes umfasst:

- Empfangen von Daten, die auf ein Genexpressionsprofil für jedes von fünf oder mehr, beispielsweise 5, 6, 7, 8, 9, 10, 11, 12, 13 oder allen Immunabwehrreaktionsgenen hinweisen, die aus der Gruppe ausgewählt sind, die aus AIM2, APOBEC3A, CIAO1, DDX58, DHX9, IFI16, IFIH1, IFIT1, IFIT3, LRRFIP1, MYD88, OAS1, TLR8 und ZBP1 besteht, wobei die Genexpressionsprofile in einer vom Patienten erhaltenen biologischen Probe bestimmt werden,
- Bestimmen der Vorhersage der Strahlentherapiereaktion basierend auf den Genexpressionsprofilen für die fünf oder mehr Gene der Immunabwehrreaktion und
- optional Bereitstellen der Vorhersage oder einer auf der Vorhersage basierenden Therapieempfehlung an eine medizinische Fachkraft oder den Patienten.

14. Diagnosekit zur Vorhersage der Reaktion eines Prostatakrebspatienten auf eine Strahlentherapie, umfassend:

- mindestens fünf Primer und/oder Sonden zur Bestimmung des Genexpressionsprofils für jedes von fünf oder mehr, beispielsweise 5, 6, 7, 8, 9, 10, 11, 12, 13 oder allen, Genen der Immunabwehrreaktion, ausgewählt aus der Gruppe bestehend aus AIM2, APOBEC3A, CIAO1, DDX58, DHX9, IFI16, IFIH1, IFIT1, IFIT3, LRRFIP1, MYD88, OAS1, TLR8 und ZBP1, in einer vom Patienten erhaltenen biologischen Probe, und
- Einrichtung nach Anspruch 12 oder ein Computerprogrammprodukt, wie in Anspruch 13 definiert.

15. Verwendung des Kits nach Anspruch 14 in einem Verfahren zur Vorhersage der Reaktion eines Prostatakrebspatienten auf eine Strahlentherapie.

16. Verfahren zur Vorhersage der Reaktion eines Prostatakrebspatienten auf eine Strahlentherapie, umfassend:

- Empfangen einer biologischen Probe, die von einem Prostatakrebspatienten erhalten wurde,
- Verwenden des Kits nach Anspruch 14 zum Bestimmen eines Genexpressionsprofils für jedes von fünf oder mehr, beispielsweise 5, 6, 7, 8, 9, 10, 11, 12, 13 oder allen, Genen der Immunabwehrreaktion, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1 in der vom Patienten erhaltenen biologischen Probe.

17. Verwendung eines Genexpressionsprofils für jedes von fünf oder mehr, beispielsweise 5, 6, 7, 8, 9, 10, 11, 12, 13 oder allen, Genen der Immunabwehrreaktion, ausgewählt aus der Gruppe bestehend aus: AIM2, APOBEC3A, CIAO1, DDX58, DHX9, IFI16, IFIH1, IFIT1, IFIT3, LRRFIP1, MYD88, OAS1, TLR8 und ZBP1, in einem Verfahren zur Vorhersage einer Reaktion eines Prostatakrebspatienten auf eine Strahlentherapie, umfassend:

- Bestimmen der Vorhersage der Strahlentherapiereaktion durch einen Prozessor basierend auf den Genexpressionsprofilen für die fünf oder mehr Gene der Immunabwehrreaktion und
- optional Bereitstellen der Vorhersage oder einer auf der Vorhersage basierenden Therapieempfehlung an eine medizinische Fachkraft oder den Patienten.

**Revendications**

1. Procédé de prédiction d'une réponse d'un sujet atteint d'un cancer de la prostate à une radiothérapie, comprenant :

- la détermination d'un profil d'expression génique pour chacun des cinq ou plus, par exemple, 5, 6, 7, 8, 9, 10, 11, 12, 13 ou tous, gènes de réponse de défense immunitaire choisis dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, lesdits profils d'expression génique étant déterminés dans un échantillon biologique obtenu à partir du sujet,
- la détermination de la prédiction de la réponse à la radiothérapie sur la base des profils d'expression génétique pour les cinq gènes ou plus de réponse de défense immunitaire, et
- facultativement, la fourniture de la prédiction ou d'une recommandation thérapeutique sur la base de la prédiction à un soignant médical ou au sujet.

2. Procédé de prédiction d'une réponse d'un sujet atteint d'un cancer de la prostate à une radiothérapie, comprenant :

- la réception du résultat d'une détermination d'un profil d'expression génique pour chacun des cinq ou plus, par exemple, 5, 6, 7, 8, 9, 10, 11, 12, 13 ou tous, gènes de réponse de défense immunitaire choisis dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, lesdits profils d'expression génique étant déterminés dans un échantillon biologique obtenu à partir du sujet,

- la détermination, par un processeur, de la prédiction de la réponse à la radiothérapie sur la base des profils d'expression génétique pour les cinq gènes ou plus de réponse de défense immunitaire, et

- facultativement, la fourniture de la prédiction ou une recommandation thérapeutique sur la base de la prédiction à un soignant médical ou au sujet.

3. Procédé de prédiction d'une réponse d'un sujet atteint d'un cancer de la prostate à une radiothérapie, comprenant :

- la détermination ou la réception du résultat d'une détermination d'un profil d'expression génique pour chacun des cinq ou plus, par exemple, 5, 6, 7, 8, 9, 10, 11, 12, 13 ou tous, gènes de réponse de défense immunitaire choisis dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, lesdits profils d'expression génique étant déterminés dans un échantillon biologique obtenu à partir du sujet,

- la détermination, par un processeur, de la prédiction de la réponse à la radiothérapie sur la base des profils d'expression génétique pour les cinq gènes ou plus de réponse de défense immunitaire, et

- facultativement, la fourniture de la prédiction ou d'une recommandation thérapeutique sur la base de la prédiction à un soignant médical ou au sujet.

4. Procédé tel que défini dans la revendication 1, 2 ou 3, dans lequel les cinq gènes de réponse de défense immunitaire ou plus comprennent six gènes de réponse de défense immunitaire ou plus.

5. Procédé tel que défini dans l'une quelconque des revendications 1-3, dans lequel les cinq gènes de réponse de défense immunitaire ou plus comprennent neuf gènes de réponse de défense immunitaire ou plus.

6. Procédé tel que défini dans l'une quelconque des revendications 1 à 5, dans lequel les cinq gènes de réponse de défense immunitaire ou plus comprennent douze gènes de réponse de défense immunitaire ou plus, de préférence, tous les gènes de réponse de défense immunitaire.

7. Procédé tel que défini dans l'une quelconque des revendications 1 à 6, dans lequel la détermination de la prédiction de la réponse à la radiothérapie comprend une combinaison des profils d'expression génique pour cinq ou plus, par exemple, 5, 6, 7, 8, 9, 10, 11, 12, 13 ou tous, des gènes de réponse de défense immunitaire avec une fonction de régression qui a été dérivée d'une population de sujets atteints de cancer de la prostate.

8. Procédé tel que défini dans l'une quelconque des revendications 1-3, dans lequel la détermination de la prédiction de la réponse à la radiothérapie comprend la combinaison des profils d'expression génique pour cinq ou plus, par exemple, 5, 6, 7, 8, 9, 10, 11, 12, 13 ou tous, des gènes de réponse de défense immunitaire avec une fonction de somme qui additionne des profils d'expression génique discrétisés des cinq gènes de réponse de défense immunitaire ou plus, dans lequel les profils d'expression génique sont discrétisés en utilisant des seuils respectifs qui ont été dérivés d'une population de sujets atteints d'un cancer de la prostate.

9. Procédé tel que défini dans l'une quelconque des revendications 1 à 8, dans lequel l'échantillon biologique est obtenu à partir du sujet avant le début de la radiothérapie.

10. Procédé tel que défini dans l'une quelconque des revendications 1 à 9, dans lequel la radiothérapie est une radiothérapie radicale ou une radiothérapie de rattrapage.

11. Procédé tel que défini dans l'une quelconque des revendications 1 à 10, dans lequel la prédiction de la réponse à la radiothérapie est négative ou positive pour l'efficacité de la radiothérapie, dans lequel une thérapie est recommandée sur la base de la prédiction et, si la prédiction est négative, la thérapie recommandée comprend une ou plusieurs parmi (i) une radiothérapie administrée plus tôt que la norme ; (ii) une radiothérapie avec une dose de rayonnement accrue ; (iii) une thérapie adjuvante, telle qu'une thérapie par privation d'androgènes ; et iv) une thérapie alternative qui n'est pas une radiothérapie.

12. Appareil permettant de prédire la réponse d'un sujet atteint d'un cancer de la prostate à une radiothérapie, comprenant :

- une entrée adaptée pour recevoir des données indiquant un profil d'expression génique pour chacun des cinq ou plus, par exemple, 5, 6, 7, 8, 9, 10, 11, 12, 13 ou tous, gènes de réponse de défense immunitaire choisis dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, lesdits profils d'expression génique étant déterminés dans un échantillon biologique obtenu à partir du sujet,
- un processeur adapté pour déterminer la prédiction de la réponse à la radiothérapie sur la base des profils d'expression génique pour les cinq gènes ou plus de réponse de défense immunitaire, et
- facultativement, une fourniture d'une unité adaptée pour fournir la prédiction ou une recommandation thérapeutique sur la base de la prédiction à un soignant médical ou au sujet.

13. Produit de programme informatique comprenant des instructions qui, lorsque le programme est exécuté par un ordinateur, amènent l'ordinateur à réaliser le procédé comprenant :

- la réception de données indiquant un profil d'expression génique pour chacun des cinq ou plus, par exemple, 5, 6, 7, 8, 9, 10, 11, 12, 13 ou tous, gènes de réponse de défense immunitaire choisis dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, lesdits profils d'expression génique étant déterminés dans un échantillon biologique obtenu à partir du sujet,
- la détermination de la prédiction de la réponse à la radiothérapie sur la base des profils d'expression génique pour les cinq gènes ou plus de réponse de défense immunitaire, et
- facultativement, la fournitude de la prédiction ou d'une recommandation thérapeutique sur la base de la prédiction à un soignant médical ou au sujet.

14. Kit de diagnostic permettant de prédire une réponse d'un sujet atteint d'un cancer de la prostate à une radiothérapie, comprenant :

- au moins cinq amorces et/ou des sondes permettant de déterminer le profil d'expression génique de chacun des cinq ou plus, par exemple 5, 6, 7, 8, 9, 10, 11, 12, 13 ou tous, gènes de réponse de défense immunitaire sélectionnés dans le groupe constitué par AIM2, APOBEC3A, CIAO1, DDX58, DHX9, IFI16, IFIH1, IFIT1, IFIT3, LRRFIP1, MYD88, OAS1, TLR8 et ZBP1, dans un échantillon biologique obtenu à partir du sujet, et
- un appareil tel que défini dans la revendication 12 ou un produit de programme informatique tel que défini dans la revendication 13.

15. Utilisation du kit tel que défini dans la revendication 14 dans un procédé de prédiction d'une réponse d'un sujet atteint d'un cancer de la prostate à une radiothérapie.

16. Procédé de prédiction d'une réponse d'un sujet atteint d'un cancer de la prostate à une radiothérapie, comprenant :

- la réception d'un échantillon biologique obtenu à partir d'un sujet atteint d'un cancer de la prostate,
- l'utilisation du kit tel que défini dans la revendication 14 pour déterminer un profil d'expression génique pour chacun des cinq ou plus, par exemple, 5, 6, 7, 8, 9, 10, 11, 12, 13 ou tous, gènes de réponse de défense immunitaire sélectionnés dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, dans l'échantillon biologique obtenu à partir du sujet.

17. Utilisation d'un profil d'expression génique pour chacun des cinq ou plus, par exemple 5, 6, 7, 8, 9, 10, 11, 12, 13 ou tous, gènes de réponse immunitaire sélectionnés dans le groupe constitué par : AIM2, APOBEC3A, CIAO1, DDX58, DHX9, IFI16, IFIH1, IFIT1, IFIT3, LRRFIP1, MYD88, OAS1, TLR8 et ZBP1, dans un procédé de prédiction de la réponse d'un sujet atteint d'un cancer de la prostate à une radiothérapie, comprenant :

- la détermination, par un processeur, de la prédiction de la réponse à la radiothérapie sur la base des profils d'expression génétique pour les cinq gènes ou plus de réponse de défense immunitaire, et
- facultativement, la fourniture de la prédiction ou d'une recommandation thérapeutique sur la base de la prédiction à un soignant médical ou au sujet.

S100 → ( START )

S102 → | OBTAIN BIOLOGICAL SAMPLES FROM FIRST SET OF PATIENTS |

S104 → | OBTAIN GENE EXPRESSION PROFILES FOR BIOLOGICAL SAMPLES |

S106 → | GENERATE REGRESSION OR SUM FUNCTION |

S108 → | OBTAIN BIOLOGICAL SAMPLE FROM PATIENT |

S110 → | OBTAIN GENE EXPRESSION PROFILE FOR BIOLOGICAL SAMPLE |

S112 → | COMPUTE PREDICTION FOR PATIENT WITH REGRESSION OR SUM FUNCTION |

S114 → | PROVIDE THERAPY RECOMMENDATION FOR THE PATIENT BASED ON THE PREDICTION |

S116 → ( END )

# FIG. 1

FIG. 2

FIG. 3

FIG. 4

EP 4 114 980 B1

FIG. 5

FIG. 6

EP 4 114 980 B1

FIG. 7

EP 4 114 980 B1

FIG. 8

FIG. 9

FIG. 10

FIG. 11

FIG. 12

FIG. 13

FIG. 14

FIG. 15

EP 4 114 980 B1

FIG. 16

FIG. 17

FIG. 18

FIG. 19

FIG. 20

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 2019028285 A2


## Non-patent literature cited in the description

- **BRAY F. et al.** Global cancer statistics 2018: GLOBOCAN estimates of incidence and mortality worldwide for 36 cancers in 185 countries. *CA Cancer J Clin*, 2018, vol. 68 (6), 394-424
- **GRIMM P. et al.** Comparative analysis of prostate-specific antigen free survival outcomes for patients with low, intermediate and high risk prostate cancer treatment by radical therapy. Results from the Prostate Cancer Results Study Group. *BJU Int*, 2012 (1), 22-29
- **DAL PRA A. et al.** Contemporary role of postoperative radiotherapy for prostate cancer. *Transl Androl Urol, Vo.*, 2018, vol. 7 (3), 399-413
- **HERRERA F.G. ; BERTHOLD D.R.** Radiation therapy after radical prostatectomy: Implications for clinicians. *Front Oncol*, 2016, vol. 6 (117)
- **PISANSKY T.M. et al.** Salvage radiation therapy dose response for biochemical failure of prostate cancer after prostatectomy - A multi-institutional observational study. *Int J Radiat Oncol Biol Phys*, 2016, vol. 96 (5), 1046-1053
- **RESNICK M.J. et al.** Long-term functional outcomes after treatment for localized prostate cancer. *N Engl J Med*, 2013, vol. 368 (5), 436-445
- **HEGARTY S.E. et al.** Radiation therapy after radical prostatectomy for prostate cancer: Evaluation of complications and influence of radiation timing on outcomes in a large, population-based cohort. *PLoS One*, 2015, vol. 10 (2)
- **PARAVATI A.J. et al.** Variation in the cost of radiation therapy among medicare patients with cancer. *J Oncol Pract*, 2015, vol. 11 (5), 403-409
- **HALL W.A. et al.** Biomarkers of outcome in patients with localized prostate cancer treated with radiotherapy. *Semin Radiat Oncol*, 2016, vol. 27, 11-20
- **RAYMOND E. et al.** An appraisal of analytical tools used in predicting clinical outcomes following radiation therapy treatment of men with prostate cancer: A systematic review. *Radiat Oncol*, 2017, vol. 12 (1), 56
- **ZHAO S.G. et al.** Development and validation of a 24-gene predictor of response to postoperative radiotherapy in prostate cancer: a matched, retrospective analysis. *The Lancet Oncology*, 2016, vol. 17 (11), 1612-1620
- **MANTOVANI A. et al.** Cancer-related inflammation. *Nature*, 2008, vol. 454 (7203), 436-444
- **GIRALDO N.A. et al.** The clinical role of the TME in solid cancer. *Br J Cancer*, 2019, vol. 120 (1), 45-53
- **SHIAO S.L. et al.** Regulation of prostate cancer progression by tumor microenvironment. *Cancer Lett*, 2016, vol. 380 (1), 340-348
- **BARKER H.E. et al.** The tumor microenvironment after radiotherapy: Mechanisms of resistance or recurrence. *Nat Rev Cancer*, 2015, vol. 15 (7), 409-425
- **GASSER S. et al.** Sensing of dangerous DNA. *Mechanisms of Aging and Development*, 2017, vol. 165, 33-46
- **ALVES DE INDA M. et al.** Validation of Cyclic Adenosine Monophosphate Phosphodiesterase-4D7 for its Independent Contribution to Risk Stratification in a Prostate Cancer Patient Cohort with Longitudinal Biological Outcomes. *Eur Urol Focus*, 2018, vol. 4 (3), 376-384
- **TILKI D. et al.** External validation of the European Association of Urology Biochemical Recurrence Risk groups to predict metastasis and mortality after radical prostatectomy in a European cohort. *Eur Urol*, 2019, vol. 75 (6), 896-900
- **COOPERBERG M.R. et al.** The CAPRA-S score: A straightforward tool for improved prediction of outcomes after radical prostatectomy. *Cancer*, 2011, vol. 117 (22), 5039-5046